(19) Europäisches Patentamt
European Patent Office
Office européen des brevets

(11) EP 1 383 902 B1

(12) # FASCICULE DE BREVET EUROPEEN

(45) Date de publication et mention de la délivrance du brevet:
**02.03.2011 Bulletin 2011/09**

(21) Numéro de dépôt: **02759816.8**

(22) Date de dépôt: **04.04.2002**

(51) Int Cl.:
*C12N 15/82* (2006.01) *C12N 15/60* (2006.01)
*A01H 5/00* (2006.01)

(86) Numéro de dépôt international:
**PCT/FR2002/001173**

(87) Numéro de publication internationale:
**WO 2002/081714 (17.10.2002 Gazette 2002/42)**

(54) **SUREXPRESSION DE LA PHOSPHOENOLPYRUVATE CARBOXYLASE**

ÜBEREXPRESSION VON PHOSPHOENOLPYRUVAT-CARBOXYLASE

OVEREXPRESSION OF PHOSPHOENOLPYRUVATE CARBOXYLASE

(84) Etats contractants désignés:
**AT BE CH CY DE DK ES FI FR GB GR IE IT LI LU MC NL PT SE TR**

(30) Priorité: **04.04.2001 FR 0104602**
**16.11.2001 FR 0114822**

(43) Date de publication de la demande:
**28.01.2004 Bulletin 2004/05**

(73) Titulaires:
- **Biogemma**
  **75001 Paris (FR)**
- **CENTRE NATIONAL DE LA RECHERCHE SCIENTIFIQUE (CNRS)**
  **75016 Paris (FR)**

(72) Inventeurs:
- **VIDAL, Jean**
  **F-91940 Gometz Le Chatel (FR)**
- **JEANNEAU, Matthieu**
  **F-36100 Saint Valentin (FR)**
- **PEREZ, Pascual**
  **F-63450 Chanonat (FR)**
- **GERENTES, Denise**
  **F-63450 Le Crest (FR)**

(74) Mandataire: **Warcoin, Jacques et al**
**Cabinet Régimbeau**
**20, rue de Chazelles**
**75847 Paris Cedex 17 (FR)**

(56) Documents cités:
**EP-A- 0 212 649** **EP-A- 0 874 056**
**EP-A- 0 916 725** **WO-A-00/08187**
**WO-A-00/28017** **JP-A- 9 107 975**

- **DATABASE WPI Section Ch, Week 199720 Derwent Publications Ltd., London, GB; Class C06, AN 1997-220420 XP002185462 & JP 09 065886 A (JAPAN TAFU GURASU KK), 11 mars 1997 (1997-03-11)**
- **COUTOUR-ANSEL D ET AL: "Effect of water stress on pyruvate, P-i dikinase and phosphoenolpyruvate carboxylase activities in the leaves of two cultivars of sorghum (Sorghum bicolor L.)." JOURNAL OF AGRONOMY AND CROP SCIENCE, vol. 176, no. 1, 1996, pages 59-69, XP008013552 ISSN: 0931-2250**
- **RODRIGUEZ-PENAGOS MIREYA ET AL: "Response of phosphoenolpyruvate carboxylase from maize leaves to moderate water deficit." JOURNAL OF PLANT PHYSIOLOGY, vol. 155, no. 4-5, octobre 1999 (1999-10), pages 631-638, XP008013560 ISSN: 0176-1617**
- **TAGU D ET AL: "TRANSCRIPTION OF A SORGHUM PHOSPHOENOLPYRUVATE CARBOXYLASE GENE IN TRANSGENIC TOBACCO LEAVES MATURATION OF MONOCOT PRECURSOR MESSENGER RNA BY DICOT CELLS" PLANT CELL REPORTS, vol. 9, no. 12, 1991, pages 688-690, XP008013498 ISSN: 0721-7714**
- **CRETIN C ET AL: "PRODUCTION IN ESCHERICHIA-COLI OF ACTIVE SORGHUM PHOSPHOENOLPYRUVATE CARBOXYLASE WHICH CAN BE PHOSPHORYLATED" PLANT MOLECULAR BIOLOGY, vol. 17, no. 1, 1991, pages 83-88, XP002230266 ISSN: 0167-4412**
- **DAI Z. ET AL: 'C4 Photosynthesis: the CO2-concentrating mechanism and photorespiration' PLANT PHYSIOLOGY vol. 103, 1993, pages 83 - 90**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

- PubMed PMID:9225854. Abstract of Ernst et al. Plant Mol. Biol. 34(3):427-443
- Pubmed abstract of Westhoff, p. et al., Ann Bot. (Lond)93(1):13-23(2004)
- HUDSPETH R.L. ET AL: 'Expression of maize phosphoenolpyruvate carboxylase in transgenic tobacco' PLANT PHYSIOLOGY vol. 98, 1992, pages 458 - 464
- DATABASE EMBL [en ligne] 23 Novembre 1989 'Maize mRNA for phosphoenolpyruvate carboxylase (EC 4.1.1.31)' Database accession no. X15238
- DATABASE WPI Week 199727, Derwent Publications Ltd., London, GB; Class C06, AN 1997-292468 & JP 9 107 975 A (MITSUI TOATSU CHEM INC) 28 Avril 1997
- IPDL MACHINE TRANSLATION OF JP9107975
- CRETIN C. ET AL: 'Complete cDNA sequence of sorghum phosphoenolpyruvate carboxylase involved in C4 photosynthesis' NUCELIC ACIDS RESEARCH vol. 18, no. 3, 1990, page 658
- CRÉTIN C. ET AL: 'The phosphoenolpyruvate carboxylase gene family of Sorghum: promoter structures, amino acid sequences and expression of genes' GENE vol. 99, pages 87 - 94
- LEPINIEC L. ET AL: 'Complete coding sequence of sorghum gene coding for the phosphoenolpyruvate carboxylase involved in C4 biosynthesis' PLANT MOLECULAR BIOLOGY vol. 19, 1992, pages 339 - 342
- LEPINIEC L. ET AL: 'Sorghum phosphoenolpyruvate carboxylase gene family: structure, function and molecular evolution' PLANT MOLECULAR BIOLOGY vol. 21, 1993, pages 487 - 502
- JEANNEAU M. ET AL: 'Improvement in drought tolerance in maize: towards the functional validation of the Zm-Asr1 gene and increase of water use efficiency by over-expressing C4-PEPC' BIOCHIMIE vol. 84, 2002, pages 1127 - 1135

LISTE DE SEQUENCES

<110> Biogemma - Centre National de la Recherche Scientifique CNRS

<120> SUREXPRESSION DE LA PHOSPHOENOLPYRUVATE CARBOXYLASE CHEZ LE MAIS

<130> D19518

<150> FR 01 04 602
<151> 2001-04-04

<150> FR 01 14 822
<151> 2001-11-16

<160> 2

<170> PatentIn Ver. 2.1

<210> 1
<211> 3163
<212> ADN
<213> Sorghum vulgare

<220>
<223> Séquence ADNc PEPC

<400> 1

```
ccatggcgtc cgagcggcac cactccatcg acgcgcagct ccgtgccctc gcacccggca 60
aggtctccga ggagctcatc cagtatgacg ccctgctcgt cgaccgcttc ctcgacatcc 120
tccaggacct acatggcccc agccttcgcg aatttgtcca ggagtgctac gaggtgtcgg 180
ccgactacga gggcaagaaa gacacgtcca agctggggga gctgggagcc aagctgacgg 240
ggctggcccc cgccgacgcc atcctggtgg cgagctccat cctgcacatg ctcaacctcg 300
ccaacctggc ggaggaagtg gagctggcgc accgccgccg gaacagcaag ctcaagcacg 360
gggacttctc cgacgagggc tccgccacca ccgagtccga catcgaggag acgctcaagc 420
gcctcgtgtc gctcggcaag acccccgcgg aggtgttcga ggcgctcaag aaccagagcg 480
tcgacctcgt cttcaccgcg catcccacgc agtccgccag gaggtcgctc ctgcagaaaa 540
acgccaggat ccggaattgt ctgacgcagc tgagtgccaa ggacgtcacg gtcgaagaca 600
agaaggagct cgacgaggct ctgcacagag agatccaagc agctttcaga actgatgaaa 660
tcaggagagc acaacccacc ccacaggatg aaatgcgcta tgggatgagc tacatccatg 720
aaactgtatg gaacggtgtg cctaagtttt tgcgccgtgt ggatacagcc ctgaagaata 780
tcggcatcaa tgagcgcctt ccctacgatg ttcctctcat taagttctgt tcttggatgg 840
gtggtgaccg tgatggaaat ccaagagtta ctccggaggt gacaagagat gtgtgcttgc 900
tgtctagaat gatggctgca aacttgtaca tcaatcaggt cgaagacctg atgtttgagc 960
tctctatgtg gcgctgcaat gatgagcttc gtgctcgagc cgaagaagtc cagagtactc 1020
cagcttcaaa gaaagttacc aagtattaca tagaattctg gaagcaaatt cccccaaacg 1080
agccctaccg ggtgatcctt ggtgctgtaa gggacaagtt atacaacaca cgcgagcgtg 1140
cacgccatct gctggcaact ggattttctg aaatttctga ggacgcggta tttaccaaga 1200
tcgaagagtt ccttgagccc cttgagctgt gctacaaatc cctgtgtgag tgcggcgaca 1260
aggccatcgc cgacgggagc ctcctggacc tccttcgcca ggtgttcacg ttcggtctct 1320
ccctggtgaa gctggacatc cggcaggagt cggagcggca gaccgacgtg atcgacgcca 1380
tcaccacgca cctcggcatc gggtcgtacc gctcgtggcc cgaggacaag cggatggagt 1440
ggctggtgtc ggagctgaaa ggcaagcgcc cactgctgcc cccggacctt cccatgaccg 1500
aggagatcgc cgacgtcatc ggcgccatgc gcgtcctggc cgagctcccg atcgacagct 1560
tcggccccta catcatctcc atgtgcacgg cgccctcgga cgtgctcgcc gtcgagctcc 1620
tgcagcgcga gtgtggcatt cgccagacgc tccccgtggt gccgctgttc gagaggctgg 1680
ccgacctgca ggcggcgccg gcgtccgtgg agaagctctt ctccactgac tggtacatca 1740
accacatcaa cggcaagcag caggtgatgg tcggctactc cgactccggc aaggacgccg 1800
gccgcctgtc cgcggcgtgg cagctgtacg tggcgcagga ggagatggcc aaggtggcca 1860
agaagtacgg cgtgaagctg accttgttcc acggccgcgg tggcaccgtc ggcaggggcg 1920
gtggcccgac gcacctcgcc atcctgtccc agccgccgga caccatcaac gggtccatcc 1980
```

```
gtgtgacggt gcagggcgag gtcatcgagt tcatgttcgg ggaggagaac ctgtgcttcc 2040
agtctctgca gcggttcacg gccgccacgc tggagcacgg catgcacccg ccggtgtctc 2100
ccaagcccga gtggcgcaag ctcatggagg agatggccgt cgtcgccacg gaggagtacc 2160
gctccgtcgt cgtcaaggag ccgcgattcg tcgagtactt cagatcggct acccctgaga 2220
ctgagtacgg gaagatgaac atcggcagca ggccagccaa gaggaggccg ggcggcggca 2280
tcaccaccct gcgtgccatc ccctggatct tctcgtggac acagacgagg ttccacctcc 2340
ccgtgtggct gggagtcggc gccgccttca agtgggccat cgacaaggac atcaagaact 2400
tccagaagct caaggagatg tacaacgagt ggccattctt cagggtcacc ctggacctgc 2460
tggagatggt tttcgccaag ggagaccctg gcattgccgg cttgtacgac gagctgcttg 2520
tcgccgagga actcaagccc tttgggaagc agctcaggga caaatacgtg gagacacagc 2580
agcttctcct acagatcgct gggcacaagg acattcttga aggcgatcct tacctgaagc 2640
aggggctgcg tctgcgcaat ccctacatca ccaccctgaa cgtgttccag gcctacacgc 2700
tgaagcggat aagggacccc agcttcaagg tgacgccgca gccgccgctg tccaaggagt 2760
tcgccgacga gaacaagccc gccggactgg tgaagctgaa cggcgagcga gtaccgccgg 2820
ggctggaaga cacgctcatc ctcaccatga agggtatcgc cgccggcatg cagaacaccg 2880
gctaggccgc ttccccttca ctcacctgca gagtactgca cggcaataat aatcacagct 2940
tccggatggt ggcgttttgt cagttttgga tggagatgct gaaaactgac accacctgtt 3000
ttcactatat gcatgtttat gtaatttcct cggctttggc ctctttatat ttttcactct 3060
tgttgtgaag tccaagtgga aaatcttggc atcttaaata tattgtaata atgaacatca 3120
tataatctac aaatttacta ttatgtatta aaaaaaaaaa aaa                   3163
```

<210> 2
<211> 1530
<212> ADN
<213> Sorghum vulgare

<220>
<223> Séquence promotrice PEPC

<400> 2

```
atggttgatc cagatctcga cctactcgat ctaatacatg ttgacagcaa gctgaggatc 60
gggacatgta ataaggagtt aggagatgtg gtatggtact aaatgcaagg tcaaaattcg 120
atgcttttc cgtgctcaac tattaactag tattattacc taattttac ttgtgatgac 180
aactaatgca tcgagccaca attcagtaaa tacttacatt aatttaagca tatgtatagt 240
atatacattt ccaattcttc tttttgtgt ggagatccac gacgatgcaa gttgctcctc 300
ccaacccaaa tccacctctc tcttaaatcc gcgtatcttc accaccacca gctgctacac 360
atcgtattgt ccaaatctgt gtcggcttga cccagtgatg tgcgcgctag atttggcagc 420
gcctgaatgc agtgcagcca cctgtatggt accccttggta gagtaacaac acccttatcc 480
ctacggcagc catgtatgac ccttatccct acggcagcca tgtataccaa tacctttctt 540
tgaaccacaa aattatagtc catatcctta accacaagtt catttttgt ttcccggtct 600
cgtaaggaaa ttaagttctg tttccacaat ttacatggat ataggacatc tatgttccta 660
acattaacat tactggataa caggcaccct ctcctccaca ccctgcaaag ccttcctcca 720
gcgccatgca tcctccgttg ctaacagaca cctctctcca catcgcgtgc aagcaaacct 780
ccaaattcta ccgatcccca gaatccggcc ttgactgcaa acagacaccc ctctccccat 840
cctgcaaacc catcagccaa ccgaataaca caagaaggca ggtgagcagt gacaaagcac 900
gtcaacagca gcaaagccaa gccaaaaacg atccaggagc aaggtgcggc cgcagctctc 960
ccggtcccct ttgcggttac cactagctaa gaatgaagat ggtactctaa atgcatactt 1020
gcgcggtttt tctctagtct aacttaataa actaaataaa caatttcttt cttatttttt 1080
taatttagtt cgtttagtta gactagagaa gaaccacgag gagttatttg aagcatcgtc 1140
cccatcctta ccactagcta gcactagcag acacccctct ccacgtcctg caaacaggca 1200
atattagcca gcggaataac acaagcaggc aagtgcgcag tgacaaagta cgtccacagc 1260
agcgatccca gccaaaagca gcgtagccac agccgcgcgc agctctcggc taccccttacc 1320
gccgatcaca tgcatgcctt tccaatcccg cgtgcacacg ccgaccacac actcgccaac 1380
tccccatccc tatttgaagc caccggccgg cgccctgcat tgatcaatca actcgcagca 1440
gaggagcagc acgagcaaca cgccgcgccg cgctccaacc atctccagct tcgttcgcgc 1500
ttcccggccc actccccggc cgccgccgcc                                  1530
```

## Description

La présente invention concerne un procédé d'amélioration de la tolérance au stress hydrique chez une plante de type $C_4$.

En conditions hydriques limitantes, les plantes $C_4$ ont su adapter leur métabolisme carboné en concentrant le milieu avoisinant la RUBISCO en $CO_2$ pour favoriser l'activité carboxylase de cet enzyme au détriment de son activité oxygénase (et son corollaire: la photorespiration), et donc le rendement photosynthétique. Ce mécanisme implique l'activité du cycle $C_4$ dont la première étape est catalysée par une carboxylase, la phosphoénolpyruvate carboxylase (PEPC EC 4.1.1.31) possédant une affinité pour le $CO_2$ (sous forme hydratée) et une vitesse catalytique fortes.

La demande de brevet européen EP 212649 et les articles de Cretin et al. (1990), et (1991a), de Lepiniec et al. (1992a; 1992b) décrivent la caractérisation de gènes codant une phosphoénolpyruvate carboxylase. Ainsi, la demande de brevet européen EP 212649 décrit le clonage d'un gène codant pour une phosphoénolpyruvate carboxylase de maïs. Les articles de Cretin et al. et de Lepiniec et al cités ci-dessus sont relatifs à l'étude du gène et/ou de l'ADNc codant une phosphoénolpyruvate carboxylase de *Sorghum.* Il faut noter que l'ARN messager de phosphoenolpyruvate carboxylase de mais (PEPC EC 4.1.1.31) est accessible sur la base de données EMBL sous le numéro X15238.

L'étude de l'expression d'un gène codant une phosphoénolpyruvate carboxylase a également été réalisée dans différents organismes. Ainsi, on peut citer la demande internationale WO 0028017 qui décrit un procédé d'obtention d'une plante $C_4$ comprenant l'introduction d'une cassette d'expression codant une phosphoénolpyruvate carboxylase bactérienne ou d'algue. L'article de Tagu et al. (1991) décrit l'étude de l'expression du gène codant la phosphoénolpyruvate carboxylase de *Sorghum Vulgare* après son introduction dans des feuilles de tabac. L'article de Cretin et al. (1991b) décrit l'étude de l'expression d'un gène codant la phosphoénolpyruvate carboxylase de *Sorghum* chez *E. Coli.* L'article de Hudspeth et al. (1992) décrit l'étude de l'expression du gène codant la phosphoénolpyruvate carboxylase de *Zea mays* dans des plants de tabac (*Nicotiana tabacum*). La demande de brevet européen EP916725 décrit l'utilisation d'un gène codant la phosphoénolpyruvate carboxylase de maïs pour produire un riz transgénique.

Les plantes $C_3$, en revanche, ne disposent pas d'un tel système de concentration du $CO_2$ et la photorespiration limite sensiblement le rendement photosynthétique. En outre, chez ces plantes, l'utilisation de l'eau est moins efficace (d'un facteur 2). Ainsi les plantes $C_4$ ont-elles, en condition de stress hydrique, un avantage sélectif par rapport aux plantes $C_3$. L'article de Contour-Ansel et al. (1996) décrit que le stress hydrique a un effet sur les activités de la pyruvate orthophosphate dikinase (PPDK) et sur la phosphoénolyruvate carboxylase de *Sorghum bicolor.* L'article de Rodriguez-Penagos (1999) décrit une augmentation de l'activité de phosphoénolyruvate carboxylase dans les feuilles de maïs en réponse à un déficit hydrique. Certains documents suggèrent l'utilisation de la PEPC d'aloès pour transformer des plantes monocotylédones afin d'augmenter l'activité phosphoénolpyruvate carboxylase chez ces plantes (WPI AN 1997-292468, la demande de brevet japonais JP9107975, IPDL traduction de JP9107975 « Machine translation »).

D'où les nombreux travaux publiés depuis 1992 visant à surexprimer chez les plantes $C_3$ une PEPC de type $C_4$ pour tenter d'augmenter la concentration en $CO_2$ au niveau de la RUBISCO et ainsi limiter la photorespiration au profit du rendement photosynthétique et de la biomasse (Hudspeth et al., 1992 ; Kogami et al., 1994 ; Gehlen et al., 1996 ; Ku et al, 1999 ; Lipka et al., 1999).

Le document référencé dans la base de données WPI AN 1997-220420 suggère d'utiliser le gène de phosphoénolpyruvate carboxykinase pour améliorer la photosynthèse des plantes $C_4$ et $C_3$.

La demande de brevet internationale WO0008187 suggère un procédé d'obtention de plantes ayant des propriétés améliorées de tolérance au stress environnemental comprenant l'introduction d'acides polynucleiques particuliers sélectionnés pour leur implication dans la résistance au stress environnemental.

Dans le cadre de la présente demande, les inventeurs se sont proposés d'apporter un concept original par rapport aux travaux précédents puisqu'il vise à surexprimer une PEPC de plante $C_4$ dans une plante $C_4$ qui en contient de façon endogène, en vue d'optimiser un métabolisme carboné déjà très efficace et notamment en condition de stress hydrique. Dans la feuille, en conditions normales, il s'agit de corriger un niveau de photorespiration par ailleurs très faible et dont l'impact attendu sur le fonctionnement photosynthétique est certainement limité en amplitude. Cependant, le stress hydrique entraîne une diminution significative de l'assimilation nette du $CO_2$, notamment par réduction de l'ouverture stomatique. Dans ces conditions de culture où l'eau et la disponibilité du $CO_2$ sont limitantes, la surexpression de la PEPC pourrait contribuer à maintenir une concentration du gaz au niveau de la RUBISCO supérieure à celle des plantes contrôles et de la sorte, une assimilation du carbone inorganique améliorée.

Par ailleurs, il existe chez toutes les plantes, $C_4$ ou $C_3$, différentes isoformes de l'enzyme équipant notamment, outre les feuilles, la racine et les grains. Une des fonctions majeures de ces isoformes est dévolue au maintien de la production d'énergie cellulaire quand des intermédiaires du cycle de Krebs (en particulier l'a-cétoglutarate) sont utilisés par d'autres voies métaboliques, comme la synthèse des acides aminés (aspartate, glutamate et acides aminés apparentés). Cette fonction, dite anaplérotique, est en particulier opérationnelle dans les grains où elle contribue au remplissage protéique (Gonzalez et al., 1998; Macnicol et al., 1998). Par ailleurs, dans cet organe, la PEPC est impliquée dans la fourniture des squelettes carbonés nécessaires à la synthèse des acides gras, ainsi à l'élaboration des réserves

lipidiques (Smith et al., 1992).

Dans le grain, la surexpression de la PEPC est donc susceptible d'améliorer significativement la capacité de remplissage protéique et lipidique, en conditions normales, si d'autres étapes limitantes des voies métaboliques concernées ne s'y opposent pas, plus sûrement en conditions de stress hydrique altérant le fonctionnement des puits et les relations source-puits de la plante. A cet égard, il convient également de souligner qu'une meilleure capacité photosynthétique (due à une surexpression de PEPC foliaire) peut avoir un impact positif sur les grains dont le remplissage dépend d'une remobilisation de l'azote et du carbone dans les feuilles lors de la maturation.

L'invention vise à pallier les inconvénients de l'art antérieur et a pour objet un procédé d'amélioration de la tolérance au stress hydrique chez une plante de type $C_4$, comprenant les étapes consistant à :

- introduire dans au moins une cellule de plante de type $C_4$ une cassette d'expression comprenant un acide nucléique codant pour une phosphoénolpyruvate carboxylase (PEPC) de type $C_4$, dont la séquence nucléotidique comprend la séquence SEQ ID No.1 ou une séquence homologue à la séquence SEQ ID No.1 identique à au moins 95% à la séquence SEQ ID No.1, sur toute la longueur de SEQ ID No.1, ledit acide nucléique codant pour un enzyme catalysant la β-carboxylation du phosphoénolpyruvate en présence de bicarbonate et d'un cation bivalent, pour former de l'oxaloacétate et du phosphate inorganique ;
- cultiver la cellule ainsi transformée de manière à régénérer une plante transgénique de type $C_4$ contenant dans son génome ladite cassette d'expression, et surexprimant ladite PEPC.

Le métabolisme carboné modifié est associé à une surexpression ou une sousexpression de la PEPC par rapport à une plante non transformée. Avantageusement le procédé concerne une surexpression de la PEPC dans les plantes transformées régénérées.

Selon un mode de réalisation le procédé comprend en outre l'identification et la sélection des cellules transformées capables de régénérer des plantes tolérantes au stress hydrique par rapport à une plante non transormée. Une telle sélection fait typiquement intervenir un gène marqueur.

Il est également divulgué ici la dite cellule transformée selon la première étape qui est également transformée par un ou plusieurs acides nucléiques choisis parmi :

- un acide nucléique codant pour un composant protéique de modulation de la chaîne de transduction et de phosphorylation de la PEPC, comme par exemple une phosphoinositol phospholipase C (PIPLC) (Coursol et al., 2000) ou une phosphoénolpyruvate carboxylase kinase (PEPCK) (Hartwell et al., 1999 ; Taybi et al., 2000);
- un acide nucléique codant pour une autre protéine du cycle $C_4$, comme par exemple la pyruvate- phosphate dikinase (PPDK) (Imaizumi et al., 1997).

Par "séquence codant pour une phosphoenolpyruvate carboxylase (PEPC), on entend une séquence codant pour un enzyme exprimé naturellement par une plante $C_4$, qui catalyse la β-carboxylation quasi-irréversible du phosphoénolpyruvate en présence de bicarbonate et d'un cation bivalent pour former de l'oxaloacétate et du phosphate inorganique (Bandurski et Greiner, 1953.). Parmi les types de PEPC retrouvés dans les plantes $C_4$, on peut citer les séquences nucléotidiques codant pour les enzymes de forme PEPC $C_3$ (fonction anaplérotique) ou PEPC $C_4$ (fonction photosynthétique + anaplérotique).

A titre d'exemple, on peut également citer préférentiellement la PEPC $C_4$ de sorgho (*Sorghum vulgare*) décrite par Crétin et al. (1990), ayant une séquence d'acides nucléiques identique ou homologue à la séquence SEQ ID N°1 du sorgho.

Avantageusement on utilisera un acide nucléique codant pour la phosphoénolpyruvate carboxylase (PEPC) de type $C_4$ de sorgho présentant une séquence nucléotidique comprenant la séquence SEQ ID N°1 sur toute la longueur de SEQ ID N°1.

Par acide nucléique, séquence nucléique ou d'acide nucléique, polynucléotide, oligonucléotide, séquence de polynucléotide, séquence nucléotidique, termes qui seront employés indifféremment dans la présente description, on entend désigner un enchaînement précis de nucléotides, modifiés ou non, permettant de définir un fragment ou une région d'un acide nucléique, comportant ou non des nucléotides non naturels, et pouvant correspondre aussi bien à un ADN double brin, un ADN simple brin que des produits de transcription desdits ADNs.

Par "séquence nucléotidique homologue", on entend toute séquence nucléotidique qui diffère de la séquence SEQ ID n° 1 par substitution, délétion, et/ou insertion d'un nucléotide ou d'un nombre réduit de nucléotides, à des positions telles que ces séquences nucléotidiques homologues codent pour des polypeptides homologues tels que définis ci-après .

Une telle séquence nucléotidique homologue est identique à au moins 95 % à la séquence SEQ ID n° 1, sur toute la longueur de SEQ ID N°1 et code pour une phosphoénolpyruvate carboxylase (PEPC) de type $C_4$.

Par « pourcentage d'identité » entre deux séquences d'acides nucléiques ou d'acides aminés au sens de la

présente invention, on entend désigner un pourcentage de nucléotides ou de résidus d'acides aminés identiques entre les deux séquences à comparer, obtenu après le meilleur alignement, ce pourcentage étant purement statistique et les différences entre les deux séquences étant réparties au hasard et sur toute leur longueur. On entend désigner par "meilleur alignement" ou "alignement optimal", l'alignement pour lequel le pourcentage d'identité déterminé comme ci-après est le plus élevé. Les comparaisons de séquences entre deux séquences d'acides nucléiques ou d'acides aminés sont traditionnellement réalisées en comparant ces séquences après les avoir alignées de manière optimale, ladite comparaison étant réalisée par segment ou par « fenêtre de comparaison » pour identifier et comparer les régions locales de similarité de séquence. L'alignement optimal des séquences pour la comparaison peut être réalisé, outre manuellement, au moyen de l'algorithme d'homologie locale de Smith et Waterman (1981, Ad. App. Math. 2 : 482), au moyen de l'algorithme d'homologie locale de Neddleman et Wunsch (1970, J. Mol. Biol. 48 : 443), au moyen de la méthode de recherche de similarité de Pearson et Lipman (1988, Proc. Natl. Acad. Sci. USA 85 : 2444), au moyen de logiciels informatiques utilisant ces algorithmes (GAP, BESTFIT, BLAST P, BLAST N, FASTA et TFASTA dans le Wisconsin Genetics Software Package, Genetics Computer Group, 575 Science Dr., Madison, WI). Afin d'obtenir l'alignement optimal, on utilise de préférence le programme BLAST, avec la matrice BLOSUM 62. On peut également utiliser les matrices PAM ou PAM250.

Le pourcentage d'identité entre deux séquences d'acides nucléiques ou d'acides aminés est déterminé en comparant ces deux séquences alignées de manière optimale dans laquelle la séquence d'acides nucléiques ou d'acides aminés à comparer peut comprendre des additions ou des délétions par rapport à la séquence de référence pour un alignement optimal entre ces deux séquences. Le pourcentage d'identité est calculé en déterminant le nombre de positions identiques pour lesquelles le nucléotide ou le résidu d'acide aminé est identique entre les deux séquences, en divisant ce nombre de positions identiques par le nombre total de positions comparées et en multipliant le résultat obtenu par 100 pour obtenir le pourcentage d'identité entre ces deux séquences.

De manière préférentielle, une telle séquence nucléotidique homologue hybride spécifiquement à la séquence complémentaire de la séquence SEQ ID n° 1, dans des conditions stringentes. Les paramètres définissant les conditions de stringence dépendent de la température à laquelle 50% des brins appariés se séparent (Tm).

Pour les séquences comprenant plus de 30 bases, Tm est définie par la relation : Tm = 81,5 + 0,41 (%G+C) + 16,6 Log (concentration en cations) - 0,63 (%formamide) . - (600/nombre de bases) (Sambmok et al, Molecular Cloning, A laboratory manual, Cold Spring Harbor laboratory Press, 1989, pages 9.54-9.62).

Pour les séquences de longueur inférieure à 30 bases, Tm est définie par la relation : Tm = 4 (G+C) + 2 (A+T).

Dans des conditions de stringence appropriées, auxquelles les séquences aspécifiques n'hybrident pas, la température d'hybridation est approximativement de 5 à 30°C, de préférence de 5 à 10°C en dessous de Tm, et les tampons d'hybridation utilisés sont de préférence des solutions de force ionique élevée telle qu'une solution 6xSSC par exemple.

Par « fragment nucléotidique », on entend tout fragment de la séquence SEQ ID n° 1, ou des séquences nucléotidiques homologues de la séquence SEQ ID n° 1, qui code(nt) pour un peptide ou une protéine présentant une activité enzymatique de phosphoénolpyruvate carboxylase, telle que définie précédemment. Ces fragments nucléotidiques présentent au moins 15 nucléotides, de préférence au moins 30, 75, 150, 300 et 450 nucléotides consécutifs de la séquence dont ils sont issus.

Par séquence nucléotidique modifiée, on entend toute séquence nucléotidique obtenue par mutagénèse selon des techniques bien connues de l'homme du métier, et comportant des modifications par rapport aux séquences normales, par exemple des mutations dans les séquences régulatrices et/ou promotrices de l'expression du polypeptide, notamment conduisant à une modification du taux d'expression ou de l'activité dudit polypeptide.

Par séquence nucléotidique modifiée, on entend également toute séquence nucléotidique codant pour un polypeptide modifié.

Les fragments nucléotidiques peuvent également être des sondes ou amorces, qui peuvent être utilisées dans des procédés de détection, d'identification, de dosage ou d'amplification de séquences nucléiques. Ces procédés peuvent faire intervenir des techniques d'amplification du type PCR (décrite par exemple dans le document US 4,683,202) ou PCR like, comme par exemple la technique SDA (Strand Displacement Amplification) ou technique d'amplification à déplacement de brin (Walker et al., 1992, Nucleic Acids Res. 20 : 1691), la technique TAS (Transcription-based Amplification System) décrite par Kwoh et al. (1989, Proc. Natl. Acad. Sci. USA, 86, 1173), la technique 3SR (Self-Sustained Sequence Replication) décrite par Guatelli et al. (1990, Proc. Natl. Acad. Sci. USA 87: 1874), la technique NASBA (Nucleic Acid Sequence Based Amplification) décrite par Kievitis et al. (1991, J. Virol. Methods, 35, 273), la technique TMA (Transcription Mediated Amplification), la technique LCR (Ligase Chain Reaction) décrite par Landegren et al. (1988, Science 241, 1077), la technique de RCR (Repair Chain Reaction) décrite par Segev (1992, Kessler C. Springer Verlag, Berlin, New-York, 197-205), la technique CPR (Cycling Probe Reaction) décrite par Duck et al. (1990, Biotechniques, 9, 142), la technique d'amplification à la Q-béta-réplicase décrite par Miele et al. (1983, J. Mol. Biol., 171, 281).

Une sonde ou amorce se définit, au sens de l'invention, comme étant un fragment d'acide nucléique simple brin ou un fragment double brin dénaturé comprenant par exemple de 12 bases à quelques kb, notamment de 15 à quelques centaines de bases, de préférence de 15 à 50 ou 100 bases, et possédant une spécificité d'hybridation dans

des conditions déterminées pour former un complexe d'hybridation avec un acide nucléique cible.

Selon un mode de réalisation de l'invention, l'acide nucléique codant pour la protéine PEPC est inséré en orientation sens dans une construction d'acide nucléique, appelée cassette d'expression ou construit, et est lié à des éléments permettant son expression et éventuellement sa régulation. La préparation d'une cassette d'expression ADN peut être réalisée de différentes manières appropriées par l'homme du métier. Un construit ADN inclut typiquement des séquences régulatrices 5' et 3' liées opérationnellement au gène de la PEPC. "Lié opérationnellement" fait référence à un lien fonctionnel entre les séquences régulatrices 5' et 3' et la séquence d'acide nucléique contrôlée. La séquence régulatrice 5' est typiquement un promoteur sélectionné. La transcription contrôlée par un promoteur lié opérationnellement produit un ARN messager fonctionnel dont la transcription produit la PEPC. Une cassette d'expression comprend typiquement une séquence d'acide nucléique pour la PEPC lié opérationnellement dans la plante transformée à une région d'initiation à la transcription (une séquence promotrice) et à une région de terminaison de la transcription. Avantageusement, la cassette d'expression peut comprendre un promoteur permettant l'expression constitutive ou ciblée de la séquence codant pour la PEPC. Parmi les promoteurs, on peut utiliser préférentiellement un promoteur constitutif tel que le promoteur actine de riz suivi de l'intron actine de riz (PAR-IAR) contenu dans le plasmide pActl-T . 609796068949642 (Mc Elroy et al., 1991) ou le promoteur 35S (Kay et al., 1987), ou un promoteur tissu spécifique. Avantageusement, on peut utiliser le promoteur High Molecular Weight Glutenin HMGW de blé (Blechl et al., 1994) ou encore le promoteur PEPC du gène de la phosphoénolpyruvate carboxylase de sorgho (Crétin et al., 1991a), qui permettent respectivement une expression de la protéine d'intérêt dans les graines ou les feuilles. Ainsi, le promoteur peut être choisi parmi le promoteur actine-intron, le promoteur HMWG ou le promoteur PEPC. Avantageusement, le promoteur est le promoteur de séquence SEQ ID N°2. On peut également utiliser des séquences promotrices induisant l'expression en condition de stress hydrique (Kasuga et al., 1999). Parmi les terminateurs utilisables dans les constructions de l'invention, on peut citer notamment l'extrémité 3' du gène de la nopaline synthétase *d'Agrobacterium tumefaciens* (Depicker et al., 1982). On peut citer également le terminateur polyA 35S du virus de la mosaïque de chou-fleur (CaMV), décrit dans l'article de Franck et al. (1980).

L'expression de la protéine PEPC peut encore être régulée par l'utilisation de séquences appropriées de type :

- introns, par exemple le 1^er^ intron du maïs adh1S (Callis et al.,1987), l'intron DSV de la mosaïque jaune du tabac (Morris et al., 1992), l'intron actine-1 du riz (McElroy et al., 1990) ;
- séquences « enhancers », par exemple l'activateur de transcription du virus de la mosaïque du tabac TEV (Carrington et Fred - 1990) ;
- séquences « leaders », par exemple le leader EMCV (Encephalomyocarditis 5' noncoding région) (elroy-Stein, O., Fuerest, T.R., et Moss B. (1989) PNAS USA, 86: 6126-6130), le leader TEV (Tobacco etch Virus) (Allison et al. (1986) ; le leader MDMV (Maize Dwarf Mozaic Virus) (Virology, 154 : 9-20) ; le leader BiP-protéine de liaison humaine (Macejack, D.G., et P. Samow (1991) Nature, 353 : 90-94), le leader AMV RNA 4 (Jobling, S.A., et Gehrke, L., (1987) Nature, 325 : 622-625), le leader TMV (Gallie, D.R. et al. (1989) Molecular Biology of RNA, pages 237-256).

Préférentiellement, la plante de type $C_4$ est le maïs.
Est également divulgué ici ladite cassette d'expression définie précédemment comprise dans un vecteur.

La cassette d'expression peut être insérée dans un vecteur nucléotidique, tel qu'un plasmide, qui peut en outre comprendre un gène marqueur, par exemple un gène permettant de sélectionner une plante transformée d'une plante qui ne contient pas l'ADN étranger transfecté. Comme gène marqueur, on peut citer un gène conférant une résistance à un antibiotique, par exemple à l'hygromycine (Herrera-Estrella et al., 1983) ou une résistance à un herbicide comme le sulfonamide asulam (WO 98/49316). On peut citer préférentiellement la séquence codante du gène Bar de *Streptomyces hygroscopicus* (N° d'accès X 17220) codant pour une Phosphinotricine Acétyltransférase qui détoxifie la phosphinotricine (agent sélectif de l'herbicide Basta/Liberty®) par acétylation (White et al., 1990).

Sont également divulguées ici les cellules végétales qui sont transformées par un vecteur tel que défini précédemment, transféré dans un hôte cellulaire susceptible d'infecter lesdites cellules végétales en permettant l'intégration dans le génome de ces dernières, des séquences nucléotidiques d'intérêt initialement contenues dans le génome du vecteur susmentionné. Avantageusement, l'hôte cellulaire peut être une souche bactérienne, telle que *Agrobacterium tumefaciens,* notamment selon la méthode décrite dans l'article d'An et al., (1986), ou encore *Agrobacterium rhizogenes,* notamment selon la méthode décrite dans l'article de Guerche et al., (1987). A titre d'exemple les cellules transformées sont typiquement des cellules de cals, d'embryons, de tissu méristématique, de cultures cellulaires en suspension.

La transformation des plantes peut être obtenue par différentes techniques appropriées connues de l'homme du métier, par exemple décrites dans Methods in Enzymology Vol. 153 ("Recombinant DNA Part D") 1987. Le terme transformation fait référence à une manipulation génétique d'une plante, d'une cellule, d'une ligne cellulaire, d'un cal, d'un tissu, d'une partie de plante ou analogue. Un tel élément est transformé en présence d'un ADN recombinant qui est introduit dans le matériel génétique de cet élément, de manière chromosomique ou extrachromosomique. L'ADN recombinant peut être un ADN étranger, un ADN hétérologue, un ADN chimère. L'ADN recombinant peut être inclus au

hasard ou de manière ciblée à un site spécifique de recombinaison homologue selon les techniques connues de l'homme du métier.

Par exemple, la transformation des cellules végétales peut être réalisée par le transfert de la région T du plasmide circulaire extra chromosomique inducteur de tumeurs Ti d'*Agrobacterium tumefaciens,* en utilisant un système binaire (Watson et al., 1994). Pour ce faire, deux vecteurs sont construits. Dans l'un de ces vecteurs, la région T a été éliminée par délétion, à l'exception des bordures gauche et droite, un gène marqueur étant inséré entre eux pour permettre la sélection dans les cellules de plantes. L'autre partenaire du système binaire est un plasmide Ti auxiliaire, plasmide modifié qui n'a plus de région T mais qui contient toujours les gènes de virulence *vir*, nécessaire à la transformation de la cellule végétale.

On peut citer la méthode décrite par Ishida et al. (1996), pour la transformation des Monocotylédones.

On peut citer aussi d'autres modes de réalisation pour introduire une cassette d'expression dans une cellule végétale, notamment les méthodes de transfert direct de gènes aux cellules végétales telles que la microinjection directes dans des embryoïdes de plante (Neuhaus et al, 1987), l'infiltration sous vide (Bechtold et al. 1993) ou l'électroporation (Chupeau et al, 1989) ou encore la précipitation directe au moyen de PEG (Schocher et al, 1986) ou le bombardement par canon de particules recouvertes de l'ADN plasmidique d'intérêt (Fromm et al., 1990).

On peut citer la méthode décrite par Finer et al. (1992), utilisant le canon à particules de tungstène ou d'or.

Le virus de la mosaïque du chou-fleur (CaMV) peut également être utilisé comme vecteur pour introduire l'acide nucléique étranger dans les cellules de plantes (Hohn et al., (1982) "Molecular Biology of Plant Tumors", Academic Press, New York, pp. 549-560 ; Howell, United States Patent N° 4,407,956). L'ADN viral CaMV est inséré dans un plasmide bactérien parent créant une molécule d'ADN recombinant qui peut être multipliée dans la bactérie. Après clonage, le plasmide recombinant peut à nouveau être cloné puis modifié par l'introduction de la séquence d'ADN souhaitée à un site de restriction unique. La portion virale modifiée du plasmide recombinant est alors coupée du plasmide bactérien parent, et utilisée pour être inoculée dans des plantes ou cellules de plantes.

L'invention a également pour objet une plante de type C4 transgénique ou partie de plante de type C4 transgénique, dans laquelle la plante est du maïs et comprend dans son génome un transgène comprenant une cassette d'expression intégrée de manière stable comprenant un acide nucléique codant pour la phosphoénolpyruvate carboxylase (PEPC) de type C4 de sorgho, dont la séquence nucléotidique comprend la séquence SEQ ID N°1 ou une séquence homologue à la séquence SEQ ID N°1 identique à au moins 95 % à la séquence SEQ ID N°1, sur toute la longueur de SEQ ID N°1, ledit acide nucléique codant pour un enzyme catalysant la β-carboxylation du phosphoénolpyruvate en présence de bicarbonate et d'un cation bivalent, pour former de l'oxaloacétate et du phosphate inorganique. Avantageusement, ledit acide nucléique codant la phosphoénolpyruvate carboxylase (PEPC) de type C4 de sorgho présente une séquence nucléotidique comprenant la séquence SEQ ID N°1 sur toute la longueur de SEQ ID N°1.

Avantageusement, ladite plante est du maïs.

Est également divulguée ici une cellule hôte transformée avec les séquences nucléiques décrites ci-dessus, ainsi qu'une plante ou partie de plante, notamment fruit, semence, grain, pollen, feuille ou tubercule, susceptibles d'être obtenues par l'un des procédés exposés précédemment. Sont cités également ici, la descendance ou un clone de telles plantes .

Il peut s'agir de plantes de grandes cultures ou potagères et fleurs, qui appartiennent aux plantes de type $C_4$, comme le maïs et le sorgho.

Il est possible d'obtenir des plantes transgéniques hybrides, par le croisement d'au moins une plante selon l'invention avec une autre.

La cassette d'expression est typiquement intégrée de manière stable dans le génome de la cellule.

Sont divulguées ici, selon un autre aspect :

- l'utilisation d'un acide nucléique codant pour une protéine PEPC pour l'obtention d'une plante transgénique de type $C_4$ à teneur modifiée en assimilats carbonés, favorisant la maturation du fruit et le remplissage de la graine ;
- l'utilisation d'un acide nucléique codant pour une protéine PEPC pour l'obtention d'une plante transgénique de type $C_4$ à teneur modifiée en assimilats carbonés, lui conférant une meilleure tolérance au stress hydrique, ledit acide nucléique étant défini tel que ci-dessus.

Un acide nucléique codant pour une protéine PEPC de type $C_4$ tel que défini ci-dessus peut être utilisé comme sonde ou amorce pour amplification pour la sélection de plantes transformées telles que décrites précédemment, présentant une meilleure résistance au stress hydrique et/ou un remplissage du grain modifié.

Est également divulguée ici, une plante présentant une augmentation de l'expression de la protéine PEPC par rapport à une plante non transformée, d'un facteur 2 à 3 par exemple, dans les feuilles et dans les grains.

La surexpression de la PEPC dans les plantes transformées permet de concentrer le $CO_2$ au site de la RUBISCO et faciliter l'assimilation du $CO_2$ malgré sa faible disponibilité en condition de stress hydrique (ouverture stomatique réduite). Les auteurs de la présente demande ont montré qu'il était possible d'améliorer l'assimilation du $CO_2$ (activité

carboxylase) en condition de stress hydrique: en effet, la surexpression de la PEPC en condition de stress hydrique et pour une résistance stomatique forte permet d'augmenter le flux de carbone photosynthétique et l'activité de la Rubisco.

Cette meilleure tolérance au stress hydrique des plantes transformées selon l'invention, par rapport aux plantes témoins, peut être mesurée par des méthodes physiologiques, morphologiques et/ ou biochimiques. A titre d'exemple, on peut citer des mesures d'activité PEPC (propriétés fonctionnelles), d'activité PEPCk et de niveau de phosphorylation de la PEPC *in planta,* des mesures d'assimilation nette en $CO_2$, de point de compensation en $CO_2$, d'efficacité photosynthétique en atmosphère appauvrie en $CO_2$, des mesures d'efficience d'utilisation en eau, et des mesures de poids frais et sec, en condition normale et de stress hydrique.

Une augmentation de l'expression de la protéine PEPC (fonction photosynthétique et anaplérotique) peut conférer aux plantes transformées une teneur modifiée en assimilats carbonés, favorisant le remplissage du grain.

Cette modification du remplissage du grain des plantes transformées selon l'invention, par rapport aux plantes non transformées, peut être mesurée par des méthodes morphologiques, physiologiques et/ou biochimiques. A titre d'exemple, on peut citer préférentiellement des techniques de spectrophotométrie infra-rouge, des techniques d'analyse élémentaire en phase gazeuse (méthode de Dumas par combustion et chromatographie en gazeuse) ou des techniques analytiques des lipides (Metcalfe et al, 1966; Bligh et Dyer, 1959).

Est également divulguée ici l'utilisation d'un acide nucléique codant pour une PEPC de type $C_4$ tel que défini ci-dessus ou un fragment de celui-ci, comme sonde ou amorce pour une amplification de type PCR, pour la sélection de plantes transformées présentant un métabolisme carboné modifié, et donc une meilleure tolérance au stress hydrique et un remplissage du grain modifié.

La séquence d'acides nucléiques codant pour une PEPC de type $C_4$ tels que définis ci-dessus, telle que celle désignée SEQ ID N°1, ainsi que tout oligonucléotide obtenu à partir de cette séquence peut ainsi être utilisée comme sonde dans des programmes de sélection assistée par marqueur, par exemple pour suivre l'introgression du gène codant pour la protéine PEPC dans une plante. Pour cela au moins une de ces sondes est marquée, par exemple par un isotope radioactif, puis mise en contact avec de l'ADN génomique de la plante, préalablement digéré par des enzymes de restriction, dans des conditions permettant l'hybridation spécifique de la sonde marquée à l'ADN en question.

Est également divulguée ici, l'utilisation d'un acide nucléique codant pour une protéine PEPC de type $C_4$ tel que défini ci-dessus ou un fragment de celui-ci, comme sonde ou amorce pour amplification, pour la sélection de plantes surexprimant naturellement une PEPC, et présentant donc une meilleure résistance au stress hydrique et/ou un remplissage du grain modifié.

D'autres avantages de l'invention apparaîtront lors de la description qui suit illustrées par les dessins dans lesquels :

La figure 1 représente une carte de restriction du plasmide pMj26 contenant le construit proPEPc~PEPC311~Nos PolyA.

La figure 2 représente une carte de restriction du plasmide pMj19 contenant le construit 35S~PEPC310~ Nos PolyA.

La figure 3 représente une carte de restriction du plasmide pWP 280 contenant le construit pActin intron~bamase~Nos PolyA.

La figure 4 représente une carte de restriction du plasmide pBIOS298 contenant le construit pActin intron-site de clonage multiple-Nos PolyA.

La figure 5 représente une carte de restriction du plasmide pMj30 contenant le construit pActin intron~PEPC3111~Nos PolyA.

La figure 6 représente une carte de restriction du plasmide pMj31 contenant le construit pHMWG~PEPC311~Nos PolyA.

La figure 7 est un diagramme représentatif de l'activité et l'immunodétection de la PEPC dans les extraits protéiques solubles de feuille de plantes T2 transformées avec le construit proPEPC-PEPC.

La figure 8 est un diagramme représentatif de l'activité et l'immunodétection de la PEPC d'extraits protéiques solubles de grains individualisés issus d'épis T2 et T3 de plantes transformées avec le construit proHMWG-PEPC.

La figure 9 représente l'efficience d'utilisation de l'eau (B) des plantes B6-F1.3.

## EXEMPLES

Tout exemple qui ne concerne pas spécifiquement l'invention revendiquée est donné à titre d'illustration.

### 1 - Construction de vecteurs recombinants

Un ADNc de PEPC de sorgho (plante $C_4$), a été isolé suivant une méthode décrite dans la littérature (Crétin et al, 1990). Le numéro d'accession de la séquence complète de l'ADNc de PEPC de forme $C_4$ de sorgho (cv Tamaran) est: X17379.

Dans un premier temps est opérée la construction de 4 vecteurs plasmidiques de base pMj-26, pMj-19, pMj-30, et pMj-31 contenant respectivement le promoteur PEPC, ou le promoteur 35S, ou le promoteur actine-intron actine (pAct), ou le promoteur HMWG, avec l'ADNc du gène PEPC dans toutes ces constructions ainsi que le terminateur de la nopaline synthétase (terNos) qui amène un signal de polyadénylation fonctionnel chez de nombreuses espèces végétales.

Des vecteurs intermédiaires sont ensuite réalisés pour la recombinaison homologue avec le vecteur pSB1 de Japan Tobacco (EP 672 752) dans *Agrobacterium tumefaciens* souche LBA 4404 (Hoekema *et al,* 1983).

Le transfert suivi de l'expression des gènes (gène de sélection et gène d'intérêt) dans le maïs est basée sur les propriétés naturelles d'*Agrobacterium tumefaciens* (Zambrisky et al. 1989) et sur la stratégie du plasmide superbinaire (Hiei *et al*; 1994 et Ishida *et al*; 1996).

Les enzymes de restriction utilisées pour les clonages sont fournies par New England Biolabs (New England Biolabs, UK). Les réactions enzymatiques sont réalisées en suivant les protocoles décrits par Sambrook et al., dans le manuel Molecular cloning (Molecular Cloning : A Laboratory Manual, 1989, Cold Spring Harbor Laboratory Press, Cold Spring Harbor, New York.

1-1 - Vecteurs plasmidiques de base

1-1-1 - Le construit pMJ-26 : pPEPC-PEPC

Le terminateur Nos (insert BamHI/HindIII de 0.4kb du pCaMVNeo (Fromm et al, 1986) est cloné dans le plasmide pUC9 (Vieira & Messing, 1982) digéré par BamHI et HindIII. Le plasmide est ouvert par BamHI et les bords rendus francs avec la polymerase Klenow. L'ADNc PEPC CP311 (insert SmaI/HindIII rendu bords francs par remplissage à la polymérase Klenow, comportant une mutation silencieuse au site NcoI ATG+120pb ; Wang et al, 1992) y est lié. L'orientation du terminateur Nos, de l'ADNc et leur jonction sont vérifiées par digestions et séquençage. L'insert NcoI de 1530bp du clone génomique CP46 (Lepiniec et al, 1992) dont la séquence complète est présentée ci-dessous en SEQ ID N°2 (séquence promotrice proPEPc) est cloné au site NcoI du plasmide précédemment obtenu. L'orientation est vérifiée par digestions et séquençage. La carte du pMJ-26 est présentée Figure.1.

1-1-2 - Le construit pMJ-19 : p35S-PEPC

L'ADNc PEPC CP310 (insert SmaI/HindIII rendu bords francs par remplissage à la polymérase Klenow, Crétin et al, 1991) est cloné au site BamHI du pCaMVNeo rendu bords francs entre le promoteur 35S (0.4kb) et le terminateur Nos (0.25kb). L'insert HindIII (3.7kb) est alors cloné au site HindIII du pSK+ (Genbank n°52325). L'orientation du promoteur 35S et du cDNA ainsi que leur jonction sont vérifiées par digestions et séquençage. La carte du pMJ-19 est présentée Figure 2.

1-1-3 - Le construit pMj-30: pactine intron- PEPc

Le clonage du construit promoteur actina~PEPC~Nos 3' utilise comme plasmide de base le plasmide pWP280 contenant la cassette pActin~intron~Barstar~Nos polyA (Figure 3), dans laquelle on remplace le fragment Barstar par le fragment PEPC.

Le plasmide de base a été obtenu selon les étapes suivantes : le gène barstar (Hartley, 1988, J. Mol. Biol., 202, 913-915) a été transféré en tant que fragment XbaI/HincII dans un site XbaI/SmaI du plasmide pW90, dérivé du plasmide pJIT30 décrit par Guérineau et al, 1990, Plant Mol. Biol., 15 :127-136) (promoteur 35SCaMV remplacé par le promoteur double 35S et la région polylinker entre les sites XbaI et EcoRI remplacée par les sites SpeI, BamHI, SmaI et PstI). La région polyA CaMV du plasmide obtenu est remplacée par la région polyA de pED23 (Dale et al., 1990, Gene, 91 :79-85) formant le plasmide pWP266. La région promotrice double 35S CaMV est enfin remplacée par le promoteur actine du riz et l'intron issu de pCOR113 (Mc Elroy et al., 1991, Mol. Gen. Genet., 231 :150-160) formant le plasmide pWP280.

Le fragment barstar a ensuite été excisé de ce plasmide pWP280 par digestion avec l'enzyme PstI, puis religaturé sur lui-même pour donner le vecteur pBIOS298 contenant le fragment promoteur actine-intron-site de clonage multiple-terminateur Nos3' (Figure 4). Le construit pBIOS-298 est ouvert au site EcoRV entre l'intron Actine et le terminateur Nos puis déphosphorylé. L'insert NcoI-EcoRV du pMJ-26 est rendu bords francs par remplissage à la polymérase Klenow puis cloné au site du pBIOS-298. L'orientation du construit a été vérifiée par digestion et la jonction IntronActine-PEPC séquencée. La carte du pMJ-30 est présentée Figure 5.

1-1-4 - Le construit pMj-31: pHMWG-PEPc

Le vecteur pBL 3214 est dérivé d'un plasmide pBluescript II SK+ (Stratagene) dans lequel on a inséré, par le choix d'enzymes de restriction spécifiques à la portée de l'homme du métier, la séquence promotrice HMWG (High Molecular Weight Glutenin) de blé (Robert et al., 1989), spécifique de l'albumen des semences et une séquence terminatrice 3' Nos de *Agrobacterium tumefaciens* (Depicker et al., 1982).

Le construit pBL 3214 est ouvert au site SmaI entre le promoteur HMWG et le terminateur Nos puis déphosphorylé. L'insert NcoI-EcoRV du pMJ-26 est rendu bords francs par remplissage à la polymérase Klenow puis cloné au site du p3214. L'orientation du construit a été vérifiée par digestion et la jonction pHMWG-PEPC séquencée. La carte du pMJ-31 est présentée Figure 6.

1-2 - Construction des vecteurs intermédiaires pour la recombinaison homologue avec pSB1 (obtention de plasmides superbinaires)

Les vecteurs servant à la recombinaison homologue dans *Agrobacterium tumefaciens* sont dérivés du vecteur pBIOS 273.

1-2-1 - Construction des plasmides pBIOS 273, 274, et 308

- *Le vecteur de base pour la recombinaison homologue est le vecteur pBIOS 2 73. Ce vecteur a été généré en 2 étapes :*
    - clonage du fragment BspDI/XhoI (pAct-Bar-terNos) du vecteur pDM 302 (Cao et al. 1992) dans les sites SmaI et BspDI du vecteur pst12 (Japan Tobacco). Le vecteur résultant de ce clonage est appelé pBIOS 272.
    - délétion du site XhoI en position 3363 du vecteur pBIOS 272 par digestion partielle avec XhoI et action de l'ADN Polymerase I, large (Klenow) fragment. Le vecteur obtenu, possédant un site unique XhoI, est nommé pBIOS 273.
- *Construction du plasmide pBIOS 274*
    - clonage du fragment (Pro A9 - Barnase - terCaMV) XhoI du vecteur pWP 128 (Paul et Al 1992) dans le vecteur pBIOS 273 restreint à XhoI
- *Construction du plasmide pBIOS 308*
    - le pBIOS 308 dérive du pBIOS 306, qui a été obtenu par clonage du fragment I/XhoI de 795 PB (ADNc AsrI) dans le vecteur pBIOS 298 précédemment décrit, restreint à ECOR V.
    - le clonage du fragment SalI/XhoI de 2970 pb du vecteur pBIOS 306 dans les sites BspDI/XhoI du vecteur pBIOS 274 génère le pBIOS 308.

1-2-2 - Génération des vecteurs intermédiaires de recombinaison contenant l'ADNc de PEPC

Ces construits ont été générés à partir des vecteurs pBIOS 274, pBIOS 273, pBIOS 308.

- *Le vecteur pBIOS 326*
  Ce vecteur a été obtenu par clonage du fragment ApaI/BstBI du vecteur pMJ-30 dans les sites PmeI/BstBI du vecteur pBIOS 308.
- *Le vecteur pBIOS 327*
  Ce vecteur a été obtenu par clonage du fragment XhoI du vecteur pMJ-31 dans les sites XhoI du vecteur pBIOS 274.
- *Le vecteur pBIOS 356*
  Ce vecteur a été obtenu par clonage du fragment SmaI/HindIII du vecteur pMJ-26 dans les sites PmeI du vecteur pBIOS 273.

2 - Transformation du maïs

2-1 - Canon à particules

La méthode utilisée repose sur l'utilisation d'un canon à particules identique à celui décrit par J. Finer (1992). Les cellules cibles sont des cellules indifférenciées en divisions rapides ayant conservé une aptitude à la régénération de plantes entières. Ce type de cellules compose le cal embryogène (dit de type II) de maïs. Ces cals sont obtenus à partir d'embryons immatures de génotype HiII selon la méthode et sur les milieux décrits par Armstrong (Maize Handbook; 1994 M. Freeling, V. Walbot Eds ; pp.665-671). Ces fragments des cals d'une surface de 10 à 20 $mm^2$ ont été disposés, 4h avant bombardement, à raison de 16 fragments par boite au centre d'une boîte de Petri contenant un milieu de culture identique au milieu d'initiation des cals, additionné de 0,2 M de mannitol + 0,2 M de sorbitol. Les plasmides décrits dans les exemples précédents et portant les séquences PEPc à introduire, sont purifiés sur colonne Qiagen$^R$ en suivant les instructions du fabricant. Ils sont ensuite précipités sur des particules de tungstène (M10) en suivant le protocole décrit par Klein (1987). Les particules ainsi enrobées sont projetées vers les cellules cibles à l'aide du canon et selon le protocole décrits par Finer (1992). Les boîtes de cals ainsi bombardées sont ensuite scellées à l'aide de Scellofrais$^R$ puis cultivées à l'obscurité à 27°C. Le premier repiquage a lieu 24h après, puis tous les quinze jours pendant 3 mois sur milieu identique au milieu d'initiation additionné d'un agent sélectif. On obtient après 3 mois ou parfois plus tôt, des cals dont la croissance n'est pas inhibée par l'agent sélectif, habituellement et majoritairement composés de cellules résultant de la division d'une cellule ayant intégré dans son patrimoine génétique une ou plusieurs copies du gène de sélection. La fréquence d'obtention de tels cals est d'environ 0,8 cal par boîte bombardée.

Ces cals sont identifiés, individualisés, amplifiés puis cultivés de façon à régénérer des plantules, en modifiant l'équilibre hormonal et osmotique des cellules selon la méthode décrite par Vain et al. (1989). Ces plantes sont ensuite acclimatées en serre où elles peuvent être croisées pour l'obtention d'hybrides ou autofécondées.

De façon préférentielle, on utilise un protocole apparenté dont le principe est décrit dans l'ouvrage Methods of Molecular Biology: Plant gene transfer and expression protocols (1995, vol. 49, PP113-123), et dans lequel les embryons immatures de génotype HiII sont directement bombardés avec des particules d'or enrobées des plasmides PEPC à introduire, préparées selon le protocole décrit par Barcelo et Lazzeri (1995). Les étapes de transformation, sélection des événements, maturation et régénération sont sensiblement analogues à celles décrites dans le protocole précédent.

2-2 - Transformation par Agrobacterium

Une autre technique de transformation utilisable dans le cadre de l'invention utilise *Agrobacterium tumefaciens,* selon le protocole décrit par Ishida et al (1996), notamment à partir d'embryons immatures prélevés 10 jours après la fécondation. Tous les milieux utilisés sont référencés dans la référence citée. La transformation débute avec une phase de co-culture où les embryons immatures des plantes de maïs sont mis en contact pendant au moins 5 minutes avec *Agrobacterium tumefaciens* LBA 4404 contenant les vecteurs superbinaires. Le plasmide superbinaire est le résultat d'une recombinaison homologue entre un vecteur intermédiaire porteur de l'ADN-T contenant le gène d'intérêt et/ou le marqueur de sélection dérivé des plasmides décrits dans les exemples précédents, et le vecteur pSB1 de Japan Tobacco (EP 672 752) qui contient : les gènes virB et virG du plasmide pTiBo542 présent dans la souche supervirulente A281 *d'Agrobacterium tumefaciens* (ATCC 37349) et une région homologue retrouvée dans le vecteur intermédiaire permettant cette recombinaison homologue. Les embryons sont ensuite placés sur milieu LSAs pendant 3 jours à l'obscurité et à 25°C. Une première sélection est effectuée sur les cals transformés : les cals embryogènes sont transférés sur milieu LSD5 contenant de la phosphinotricine à 5 mg/l et de la céfotaxime à 250 mg/l (élimination ou limitation de la contamination par *Agrobacterium tumefaciens*). Cette étape est menée 2 semaines à l'obscurité et à 25°C. La deuxième étape de sélection est réalisée par transfert des embryons qui se sont développés sur milieu LSD5, sur milieu LSD10 (phosphinotricine à 10 mg/l) en présence de céfotaxime, pendant 3 semaines dans les mêmes conditions que précédemment. La troisième étape de sélection consiste à exciser les cals de type I (fragments de 1 à 2 mm) et à les transférer 3 semaines à l'obscurité et à 25°C sur milieu LSD 10 en présence de céfotaxime.

La régénération des plantules est effectuée en excisant les cals de type I qui ont proliféré et en les transférant sur milieu LSZ en présence de phosphinotricine à 5 mg/l et de céfotaxime pendant 2 semaines à 22°C et sous lumière continue.

Les plantules ayant régénéré sont transférées sur milieu RM + G2 contenant 100mg/l d'Augmentin pendant 2 semaines à 22°C et sous illumination continue pour l'étape de développement. Les plantes obtenues sont alors transférées au phytotron en vue de leur acclimatation.

3 - Expression

3-1 - Extraction de la PEPC de feuilles et de grains de maïs.

- Les feuilles sont prélevées et immédiatement congelées dans l'azote liquide. Le broyage s'effectue dans un mortier préalablement nettoyé à l'éthanol 100% et refroidi sur glace. Un disque foliaire de 18 mm de diamètre est extrait dans 200 μL de tampon d'extraction : Tris-HCl pH 8.0, glycérol 20%, $MgCl_2$ 10 mM, EDTA 1 mM, DTT 1 mM, PVP insoluble 2% (p/v), du sable de fontainebleau et des inhibiteurs de protéases : leupeptine 2mg.$L^{-1}$ , chymostatine 2mg.$L^{-1}$, PMSF 1mM et E64 1mg.$L^{-1}$. Quand des mesures de niveau de phosphorylation de la PEPC sont programmées, les inhibiteurs de phosphatase : acide okadaïque 0.1mg.$L^{-1}$ et la micro-cystine-LR 10nM sont alors ajoutés. Le broyat est ensuite centrifugé à 4°C pendant 15 minutes à 20000g pour éliminer les débris.
- Les grains sont préalablement réduits en poudre dans un broyeur à bille (Retsch). Les protéines sont extraites par mise en suspension de 100μL de poudre dans 400 μL du tampon précédemment décrit sur glace. Ce mélange est vortexé et centrifugé à 4°C pendant 15 minutes à 20000g pour éliminer les débris.

Dans les deux cas, le surnageant constitue l'extrait brut de protéines solubles. Il peut être utilisé immédiatement pour des mesures d'activité PEPC ou congelé à l'azote liquide et conservé à -20°C pour une détection ultérieure en Western blot.

3-2 - Mesures d'expression

3-2-1 - Western blot

L'électrophorèse en gel de poly-acrylamide en présence de SDS permet de séparer les protéines en fonction de leur masse moléculaire. La technique est inspirée de Laemmli (1970).

La détection immunologique de la PEPC sur la membrane de nitrocellulose se fait après blocage par l'addition d'un anticorps primaire qui peut être:

a) un anticorps polyclonal de la PEPC $C_4$ de sorgho, préparé selon la méthode décrite dans Vidal *et al.* (1980).

b) un anticorps anti-site de phosphorylation, dirigé contre un peptide synthétique de 23 acides aminés de la séquence N-terminale de la PEPC $C_4$ de sorgho [ERHHSIDAQLRALAPGKVSEE(YG)] (Crétin et al, 1990) qui contient le site de phosphorylation. Les anticorps ont été préparés chez le lapin et purifiés par chromatographie d'affinité sur protéine A Sépharose. Ces anticorps reconnaissent avec la même efficacité les formes phosphorylées ou non de l'enzyme. La dilution utilisée est de 1/2000.

c) un anticorps anti C-terminal, dirigé contre un peptide synthétique de 20 acides aminés de la séquence C-terminale de la PEPC $C_4$ de sorgho [(Y)EDTLILTMKGLAAGMQNTG)]. Ces anticorps ont été obtenus dans des lapins et purifiés. La dilution utilisée est de 1μL/15000.

d) un anticorps monoclonal produit contre la forme $C_4$ de la PEPC de Sorgho par la technique des hybridomes. Plus de 400 hybridomes produisant des anticorps ont été produits après fusion des cellules de rate de souris immunisées, avec des cellules de myélome NS1. Les anticorps 83 et 91 ont montré une très haute spécificité pour la PEPC $C_4$ de sorgho (Thomas *et al.,* 1987).

La détection immunologique de la PEPC de Type $C_4$ de sorgho dans les plantes a permis de révéler la présence significative de la protéine transgénique, notamment avec le construit proPEPC-PEPC. En outre, cette surexpression est corrélée avec les différences d'activité observées entre les plantes transgéniques, et les plantes témoins, jusqu'à 2,2 fois le niveau de PEPC endogène au niveau des feuilles.

Le construit proHMWG-PEPC (grains) a également donné des résultats satisfaisants, car le niveau d'expression de la PEPC est jusqu'à six fois supérieur par rapport au niveau d'expression de la PEPC endogène dans les grains issus de plantes de types $C_4$ transformées.

3-2-2 - Mesures d'activité et paramètres enzymatiques

*a) - Capacité de la PEPC*

La mesure de l'activité maximale de la PEPC est effectuée à l'aide d'un spectrophotomètre (Cary 50, Varian). Le principe repose sur le couplage de deux réactions : la β-carboxylation du PEP par la PEPC produit l'oxaloacétate

qui est réduit en malate par la malate déshydrogénase (MDH) à NAD. On mesure la diminution de l'absorption à 340nm due à l'oxydation du NADH à 30°C. Le milieu réactionnel contient dans un volume final de 1mL : Hépès/KOH 100 mM, pH 8, $MgCl_2$ 5 mM, NADH 0.2 mM, $NaHCO_3$ 5 mM, PEP-Na 5 à 10 mM (Roche) et 3 unités enzymatiques de MDH à NAD (Roche). La réaction est initiée par addition de 5 à 50μL d'extrait protéique brut de feuille ou de grain contenant la PEPC. Une unité enzymatique correspond à la formation d'une μmole de produit par minute dans les conditions expérimentales définies ci-dessus.

Une étude biochimique a permis de mettre en évidence une augmentation de l'activité enzymatique maximale PEPC d'un facteur 2,2 par rapport au niveau témoin chez la feuille (Figure 7).

Pour le grain, l'activité enzymatique est multipliée jusqu'à 6 fois, entre les grains transformés et les grains non transformés à l'intérieur d'un même épi. (Figure 8). Quelle que soit la génération, la proportion des grains qui sont transformés à l'intérieur d'un même épi, issu d'un croisement entre une plante transformée et une plante non transformée ou issu d'un rétrocroisement, varie de 50 à 100 % en fonction du nombre de loci présentant le transgène. Dans la plupart de cas, cette proportion est de 50% des grains transformés à l'intérieur d'un même épi.

L'augmentation d'activité PEPC est corrélée quantitativement à la protéine transgénique (immunodétection).

Plus précisément, le niveau d'expression de l'enzyme codée par le transgène (proPEPC~PEPC) dans les maïs transformés a été déterminé par une mesure de l'activité catalytique dans des extraits protéiques foliaires, et par "Western blot".

50 plantes ont été analysées :

- 15 plantes correspondant à 6 événements de transformation par A. *tumefaciens* (A6-B, C, D, E, F, I)
- 35 plantes correspondant à 14 événements de transformation par biolistique (B6-A, B, C, D, E, F, G, H, I, J, K, L, M, N)

Des plantes (B6-T 1.98183722408633, B6-A4.2) ont été plus précisément caractérisés. Le niveau de surexpression en terme d'activité spécifique PEPC (à pH optimal et pour une concentration en PEP de 10mM) est de +112% ± 13 (doublement) pour les descendants transformés de la plante B6-T 1.98183722408633 et de +17% ± 16 pour les descendants transgéniques de la plante B6-A4.2 ; les extraits protéiques foliaires correspondants contiennent la PEPC de sorgho (immunodétection).

Les inventeurs ont constaté que les plantes ayant les niveaux de surexpression les plus élevés en protéine recombinante (B6-T .15244901723741 et B6-A4 transformées par biolistique) sont celles qui présentent le plus grand nombre de copies du transgène (de 3 à 7 copies).

Les inventeurs ont également recherché si le niveau d'activité de la PEPC $C_4$ dans les plantes de maïs transgéniques est associé à une modification du niveau d'accumulation des transcrits.

Dans un premier temps, il a été vérifié que l'ARNm était effectivement produit à partir du transgène. Par Northem blot, la sonde utilisée (65 pb en 5' de l'ADNc de PEPC $C_4$ de sorgho) ne permet pas distinguer les ARNm codant l'enzyme de maïs et de sorgho (ils sont en effet très homologues, parties 5' et 3' non traduites comprises). En revanche, l'utilisation de la technique de RT-PCR met en évidence la présence spécifique du transcrit dans les extraits foliaires d'ARN totaux des plantes présentant une modification à la hausse ou à la baisse de l'activité PEPC .

Par ailleurs, les expériences de Northem blot (avec une sonde hybridant les deux ARNm de PEPC $C_4$ produits) montrent que la quantité des transcrits totaux de PEPC $C_4$ varie corrélativement à la quantité de l'enzyme.

En outre il a été vérifié que le promoteur utilisé (proPEPC : 1530 pb en amont du gène $C_4$ de sorgho) contient l'information permettant le contrôle de l'expression spécifique du transgène dans le mésophylle des plantes transformées qui surexpriment fortement la PEPC $C_4$ de sorgho.

*b) - Détermination du $S_{0.5}$ pour le PEP et de la sensibilité au malate de la PEPC*

La constante enzymatique $S_{0.5}$ est la concentration en PEP qui détermine une vitesse de catalyse égale à 50% de la vitesse maximale.

Les résultats montrent que le $S_{0.5}$ pour le PEP de la PEPC dans les grains est significativement supérieur à celui de l'enzyme des grains contrôles, ce qui permet de mettre en évidence la présence d'une forme $C_4$ (recombinante) dans les grains transformés.

Le malate est un inhibiteur physiologique de la PEPC. L'activité de la PEPC est mesurée dans des conditions sub-optimales (PEP 3mM, pH 7,3) en présence et en absence de L-malate 1.2 mM. Les résultats sont exprimés selon une activité en présence de malate/ activité en absence de malate x 100. La forme déphosphorylée est plus sensible à l'inhibition par le malate (70%) que la forme phosphorylée (environ 30%). Ce test permet d'estimer les variations de l'état de phosphorylation de l'enzymes foliaire lorsque la plante est exposée à différents stimuli (lumière, obscurité, stress).

Des études préliminaires n'ont montré aucune différence significative de sensibilité au malate entre les plantes surexprimant beaucoup ou peu la PEPC recombinante. Ainsi la PEPC exogène serait phosphorylée dans la feuille à la lumière dans les mêmes proportions que la PEPC endogène. Cela indiquerait que sous une forte luminosité, l'augmentation d'activité PEPC n'est pas compensée par une moindre phosphorylation.

3-2-3 - Dosage du malate

L'extraction des métabolites s'obtient par broyage de 10mg de matériel (foliaire ou grain) dans 100μL d'acide perchlorique à 5% (v/v) avec du sable et 2% (p/v) de PVP insoluble. Le broyat est centrifugé à 20000g à 4°C pendant 5 minutes. Le surnageant est ajusté à un pH de 7.6 environ avec 16 μL de carbonate de potassium 50%. Le principe du dosage (kit Roche) consiste en l'oxydation du L-malate en oxaloacétate par la malate déshydrogénase (MDH) en présence de NAD. La réaction est amenée à complétion dans le sens de la formation de l'oxaloacétate en couplant cette réaction à celle de la glutamate-oxaloacétate-transaminase selon le schéma réactionnel décrit ci-dessous.

$$\text{(1) malate} + NAD^{+} \underset{}{\overset{\text{MDH}}{\rightleftarrows}} \text{oxaloacétate} + NADH + H^{+}$$

$$\text{(2) Oxaloacétate} + \text{glutamate} \overset{\text{GOT}}{\rightleftarrows} \text{aspartate} + 2\ \alpha\text{-cétoglutarate}$$

La quantité de NADH formé (1 NADH pour 1 oxaloacétate) est mesurée par spectrophotométrie à 340 nm et la formule suivante permet de calculer la concentration en L-malate (C en $g.L^{-1}$) des extraits analysés :

$$C = \Delta A \times [(V \times MW) / (\varepsilon \times d \times v \times 1000)]$$

Dans laquelle :

V = volume du milieu réactionnel en mL
v = volume de l'extrait en mL
MW = masse moléculaire du L-malate en $g.mol^{-1}$
d = longueur du trajet optique en cm
$\varepsilon$ = coefficient d'absorption spécifique du NADH à 340 nm = 6.3 $mmol^{-1} . cm^{-1}$

Aucune différence significative de la teneur en malate n'a pu être mesurée entre les grains transformés et les grains contrôles. Si du malate est surproduit dans les grains transformés, il est donc probablement métabolisé.

3-3 - Etudes moléculaires

Parmi les transformants sont préférentiellement choisis ceux qui présentent une insertion monolocus/monocopie (1 copie du gène d'intérêt et 1 copie du marqueur de sélection) sans séquence plasmidique indésirable. La technique Southem avec plusieurs enzymes de restriction et plusieurs sondes appropriées (Southern, 1975) peut notamment être utilisée pour identifier et caractériser l'insertion dans le génome de la plante, permettant ainsi de différencier les événements de transformation. Cette méthodologie permet en effet de mettre en évidence des différences individuelles dans la taille des fragments de restriction obtenus avec une enzyme donnée et une sonde donnée, correspondant à des emplacements définis sur le génome.

On peut utiliser les stratégies suivantes:

- pro HMWG pBIOS 327: digestion ApaI ou EcoR V, utilisation de la sonde intron actine et d'une sonde BAR.
- proActine pBIOS 326: digestion KpnI, sonde BAR. Sur ce construit la redondance du promoteur et de l'intron actine ne facilite pas l'utilisation d'une sonde simple. Une sonde RB pourrait être utilisée comme seconde matrice d'hybridation.
- proPEPC pBIOS 356: digestion KpnI, sonde BAR et promoteur PEPC. Les profils obtenus sont ceux attendus.

4 - Mesures physiologiques des maïs en conditions limitantes en eau et en $CO_2$

4.1 Assimilation du $CO_2$, et mesures de poids sec/frais en conditions de stress hydrique

Le protocole utilisé comprend les étapes suivantes (d'après Pelleschi et al 1997):

Les grains (24) de lignées transformées sont semés sur perlite dans des pots (1 grain/pot) de 10cm de diamètre et 25 cm de haut que l'on place en serre. La température est de 26°C le jour et 18°C la nuit, l'humidité relative est de 70% et si besoin, la lumière naturelle est supplémentée d'une lumière artificielle (lampe Philips Sun-T Agro) fournissant 400 μE au minimum. La solution nutritive (Hydrocani C2) est complétée par une solution de Séquestrene (g/L) permettant de satisfaire aux besoins en fer du maïs. Un Western blot sur un extrait protéique de la seconde feuille permet de déterminer le génotype des plantes et de distribuer aléatoirement les ségrégants dans la serre. Le stress hydrique est appliqué quand 90% des plantes ont la ligule de la $4^{ème}$ feuille visible, ce qui est généralement observé environ 15 à 20 jours après semis. Les paramètres photosynthétiques sont mesurés régulièrement (appareil IRGA CIRAS I de PP Systems) sur chaque individu pendant 15 jours pour suivre l'établissement de la sécheresse sur les deux lots. En fin de manipulation le potentiel hydrique de la sixième feuille, la masse fraîche et la masse sèche des parties aériennes sont mesurées et un échantillon est congelé pour les analyses biochimiques.

La mesure des paramètres photosynthétiques dans les conditions d'expérience montrent que :

- l'assimilation nette en $CO_2$ augmenterait en moyenne jusqu'à 7% par rapport aux témoins, à la fois en condition normale, et de stress hydrique.
- en fin de stress hydrique, l'efficience d'utilisation de l'eau augmente aussi, de 25% par rapport aux témoins
- après 18 jours de stress hydrique, les poids frais et secs augmentent respectivement de 10% et 20% par rapport aux témoins.

Plus précisément, les mesures des paramètres photosynthétiques ont été effectuées sur des plantes soumises à une contrainte hydrique en serre. L'arrosage automatique a été arrêté dès que la ligule de la $4^{ème}$ feuille est apparue. Au cours de la mise en place de la contrainte hydrique, les mesures ont été effectuées à intervalles de temps réguliers. Pour une assimilation photosynthétique égale, les plantes transgéniques B6-T étudiées se distinguent très significativement des plantes contrôles quant à A/Gs (rapport assimilation nette en $CO_2$ / conductance stomatique pour l'eau) comme indiqué sur la figure 9. Sur cette figure, quand 90% des plantules ont atteint le stade $4^{ème}$ feuille ligulée l'arrosage est arrêté et les paramètres photosynthétiques sont mesurés périodiquement. Les points représentent la moyenne des mesures ( $\pm$ SE) ; plantes maintenues arrosées ($\Delta$), plantes contrôles (□), plantes transformées (●). Le degré de confiance statistique des différences observées entre les plantes transgéniques et non transgéniques est indiqué par les étoiles. * * p<0,01.

A partir du $10^{ème}$ jour de stress, ce paramètre augmente plus rapidement pour les plantes transgéniques atteignant une valeur de 21,6, soit une augmentation de 30,1% $\pm$20,5 (p<0.005). En dépit d'une hétérogénéité de germination induisant une variabilité des mesures, une deuxième série d'expériences sur 40 plantes B6-T a permis de confirmer les données préliminaires (+16%; p>0.2). Ces résultats reflètent une amélioration de la gestion de l'eau chez les plantes transgéniques soumises à une sécheresse.

Inversement, chez des plantes transgéniques A6-T 15244901723741, déficientes en PEPC $C_4$, A et A/Gs sont très inférieurs, par rapport au contrôle, au cours de l'établissement de la contrainte hydrique. En fin de stress, les plantes transgéniques montrent une diminution de A et de A/Gs respectivement de 38 % $\pm$ 13.5 (p<0.001) et 47% $\pm$ 4 (p<0.001).

4.2 Point de compensation du $CO_2$ et efficacité photosynthétique en atmosphère appauvrie en $CO_2$

La culture s'effectue à partir de deux pools de grains: les grains sont semés sur de la perlite imbibée d'une solution nutritive (adaptée au maïs) ou sur du terreau irradié et cultivés en serre.

Au stade 4 à 5 feuilles, après 8 à 10 jours de culture, le test Basta est effectué: on badigeonne la $3^{ième}$ feuille (sur une surface de 4 $cm^2$) à l'aide d'une éponge imbibée d'une solution aqueuse à 0,75 g/l de glufosinate d'ammonium.

La lecture du test Basta est effectuée 5 jours plus tard: les feuilles nécrosées révèlent les plantes qui n'ont pas été transformées, et celles ci seront utilisées ultérieurement comme référence (contrôle).

Les plantes sont ensuite transférées dans des enceintes phytotroniques et le point de compensation en $CO_2$ déterminé après 20 à 25 jours.

Les mesures d'échange de $CO_2$ sont réalisées pendant 21 jours à partir des plantes maintenues dans les conditions de culture suivantes: concentration en $CO_2$ de 50 ppm; lumière de 800 à 1000 $\mu E.m^{-2}.s^{-1}$; température 26°C jour et 20°C nuit; hygrométrie de 80% jour et nuit. Cette concentration subatmosphérique en $CO_2$ permet de mimer les

effets d'un stress hydrique et en particulier la réduction de l'ouverture stomatique.

En fin d'expérience la masse végétale fraîche et le poids sec sont déterminés sur l'ensemble des échantillons.

Des expériences ont montré que les points de compensations des plantes transgéniques (plante entière) sont inférieurs de 2 à 4 ppm (soit une réduction de 20 à 30%) à ceux des plantes non transformées.

Plus précisément des plantes soumises à l'expérience ont été cultivées en serre. Une synthèse des résultats est présentée ci-dessous.

| B6-T1 | NT | T | p | T/NT % |
|---|---|---|---|---|
| Nb de feuilles visibles 10 j après semis<br>Nb de feuilles visibles 20 j après semis | 2,48 ±0,59<br>5,58 ± 0,47 | 2,77 ± 0,42<br>5.79 ± 0,42 | p=0,04<br>p=0,11 | + 11,7*<br>+3,8 |
| Largeur feuille 4 (cm)<br>Surface feuille 4 ($cm^2$) | 2,27 ± 0,17<br>36,2 ± 6,0 | 2,44 ± 0,25<br>38,7 ±8,3 | p=0,02<br>p>0,2 | + 7,5*<br>+ 6.9 |
| Largeur feuille 5 (cm)<br>Surface feuille 5 ($cm^2$) | 3,34 ± 0.34<br>76.7 ± 18.5 | 3,56 ± 0,36<br>82,7 ± 11,8 | p=0,02<br>p>0,2 | + 6,6*<br>+ 7.8 |
| Largeur feuille 6 après 18 j de stress (cm)<br>Surface feuille 6 après 18 j de stress ($cm^2$) | 4,22 ± 0,42<br>156 ± 12,3 | 4,61 ± 0,17<br>165 ± 24,6 | p=0,01<br>p>0,2 | + 9,2**<br>+ 5,8 |
| Densité stomatique 4ème feuille (nb stomates.$mm^{-2}$)<br>Densité stomatique 10ème feuille (nb stomates.$mm^{-2}$) | 49,0 ± 5,0<br>39,3 ± 1,7 | 38,0 ± 5,3<br>36,3 ± 3 | p=0,007<br>p=0,03 | - 22**<br>-7,6* |
| Poids frais après 18 j de stress (g)<br>Poids sec après 18 j de stress (g) | 22,18 ± 1,74<br>2,77 ± 0,52 | 24,49 ± 2,53<br>3,32 ± 0,50 | p=0,03<br>p=0,02 | + 10,4*<br>+ 19,8* |

On observe que la transformation se manifeste par des effets pléiotropiques :

- Au début de la culture (10 jours après semis), les plantules B6-T 1 transgéniques possèdent une avance de croissance d'environ 0,3 feuille sur les plantes non transgéniques. Cet écart, notable en fin de phase auxotrophe, s'amenuise avec l'âge des plantes puisqu'après 20 jours de culture l'avance n'est plus que de 0,2 feuille.
- La largeur foliaire est supérieure chez les plantes transformées de respectivement 7,5% et 6,6% pour les feuilles 4 et 5 en conditions normales de culture; ce résultat est exacerbé (+9,2%) pour la feuille 6 à la fin d'un stress de 18 jours.

La teneur en $CO_2$ de l'atmosphère de culture des plantules de maïs influence la densité stomatique. Sur la 4ème et la 10ème feuille l'augmentation d'activité PEPC est corrélée à une réduction significative du nombre de stomates par unité de surface.

Enfin, l'ensemble des caractères photosynthétiques et biométriques propres aux plantes transformées se concrétisent en fin de stress par l'existence d'un surcroît significatif de poids frais (+10,4 %) ou de matière sèche (+19,8%) pour les plantes transformées par rapport aux contrôles. Cette augmentation pourrait être la résultante d'un appareil photosynthétique plus performant qui par un phénomène intégratif (exponentiel dans l'absolu) permet à la plante d'augmenter sa vitesse de croissance.

Inversement, quand la capacité de la PEPC photosynthétique est réduite de 78%, les performances photosynthétiques du maïs sont considérablement altérées (plantes A6-F1.1 déficientes en PEPC C4). Les feuilles sont moins larges (-10%), le poids sec mesuré est moins important (-30%) et le nombre de stomates a augmenté (+13.5%) par rapport aux plantes contrôles, avec un aspect plus chétif des plantes, notamment une réduction des dimensions de la gaine et de la pigmentation.

4.3 Autres mesures au champ

La résistance au stress hydrique des plantes transformées selon l'invention, par rapport aux plantes témoins, peut être appréciée à partir de diverses méthodes d'analyses phénotypique, physiologique et/ou biochimique, pour des conditions d'irrigation particulières, en conditions normales (culture traditionnelle avec arrosage) et en conditions de stress hydrique.

Les notations effectuées aux différentes périodes de floraison et de récolte consistent à mesurer l'effet de la tolérance au stress sur la production en grain, notamment : le pourcentage de fécondation (rapport du nombre de grains par épi / nombre d'ovules fécondables), le nombre de rangs par épi, le nombre de grains par rang, l'humidité des grains,

le poids de 1000 grains.

5 - Modifications quantitatives et qualitatives des teneurs en protéines et lipides.

5.1 Technique infra-rouge

L'analyse dans le proche infrarouge est une technique spectroscopique qui utilise le spectre électromagnétique naturel. La région du proche infrarouge est la zone où le spectre est défini par des longueurs d'onde comprises entre 700 nm et 2500 nm. Il est situé entre la région visible et la région de l'infrarouge moyen. La zone du proche infrarouge est idéale pour effectuer les mesures de propriétés chimiques d'échantillons liquides, solides, gazeux ou en suspension épaisse. Les appareils utilisant le proche infrarouge reposent sur des monochromateurs à grille de dispersion. Cette technique est relativement précise, rapide et non-destructive. Les molécules typiques analysées dans le NIR contiennent des liaisons CH, OH et NH et notamment les matières grasses, les protéines, l'amidon et les sucres solubles.

Des changements d'équilibre entre les différentes voies métaboliques de synthèse des acides organiques, des acides gras et des acides aminés peuvent être étudiés grâce à cette technique sur le grain entier.

5.2 Analyseur d'éléments (C, N)

La méthode d'analyse à sec des éléments (Dumas) est basée sur une transformation des particules solides en gaz par une combustion totale et très rapide dans un four à 1200°C. Tous les gaz produits sont entraînés hors du four par un flux continu de gaz non réactif (hélium). Le carbone est converti en $CO_2$ au cours de la combustion et les composés azotés en gaz $N_2$ ou différents oxydes d'azote (NOx). Les gaz passent sur une colonne de réduction composée de cuivre (600°C) dans laquelle les oxydes d'azote libèrent l'oxygène et émergent de la colonne sous forme $N_2$. L'eau est retenue sur une colonne de perchlorate de magnésium. Le mélange entre alors sur une colonne de chromatographie en phase gazeuse (Chromosorb) séparant l'azote (élué en premier) du dioxyde de carbone. La détection par catharomètre mesure la différence de conductivité entre un flux de référence (hélium) et le flux analysé.

Une modification de la teneur en protéine totale par rapport à la teneur en glucides peut être détectée par cette méthode dans un broyat de chaque grain.

5.3 Teneur en lipides (acides gras et triglycérides)

L'extraction des lipides totaux est réalisée selon la méthode de Bligh et Dyer (1959). Les broyats de grains sont fixés dans l'éthanol bouillant puis l'extraction est faite par addition d'un volume de chloroforme, puis d'eau (1/1/1 ; V/V/V). Une quantité connue d'heptadécanoate (C17:0) est ajoutée au milieu d'extraction. Cet acide gras absent du maïs peut donc être utilisé comme témoin interne.

L'extrait d'acides gras est méthylé et les esters méthyliques analysés par chromatographie en phase gazeuse. La méthylation est réalisée selon la méthode de Metcalfe et al (1966). Cette technique sépare les esters méthyliques d'acides gras selon le nombre d'atomes de carbone et le degré d'insaturation. Un intégrateur permet de calculer la quantité de chaque acide gras en fonction du standard interne.

Une modification de la teneur en lipides totaux par rapport au poids sec peut être détectée par cette méthode dans un extrait de chaque grain.

L'augmentation du flux d'oxaloacétate puis de malate (via la PEPC) vers les leucoplastes semblerait entraîner une augmentation de la teneur en acides gras.

**REFERENCES BIBLIOGRAPHIQUES**

- An et al. (1986), Plant Physiol, 81 : 86-91
- Armstrong et Al (1994), The Maize Handbook, 665-671
- Bandurski et Greiner (1953), J. Biol. Chem., 204:781-786.
- Barcelo et al. (1995), Methods of Molecular Biology, 49:113-123
- Bechtold et al. (1993), Comptes rendus Académie des Sciences Paris Serie 3,316:1194-1199
- Blechl et al. (1996), Nat Biotechnol, 14(7):875-879
- Bligh et al. (1959), Canadian journal ofbiochemestry and physiology, 37:911917
- Callis et a 1. (1987), Genes Dev.,1 : 1183
- Cao et al. (1992), Plant Cell Reports
- Chupeau et al. (1989), Biotechnology, 7(5):503-508

- Contour-Ansel et al. (1996) J. Agron. & Crop Sci. 176(1) :59-69
- Coursol et al. (2000), The Plant Journal, 23(4):497-506.
- Cretin et al. ( 1990), Nucleic Acids Res, 11; 18(3):658
- Cretin et al. (1991a), Gene, 99:87-94
- Cretin et al. (1991b), Plant Molecular Biology, 17: 83-88
- Dale et al., 1990, Gene, 91:79-85
- Depicker et al., (1982), J. Mol. Appl.Genet., 1, 561-573.
- Finer et al. (1992). Plant Cell Report, 11:323-328
- Franck et al. (1980), Cell, 21:285-294
- Fromm M. et al. (1990), Biotechnology, 8:833-839
- Gehlen et al. (1996), Plant Mol Biol, 32(5):831-848
- Gonzalez et al. (1998), Plant physiology, 116:1249-1258.
- Guerche et al. (1987), Mol. Gen. Genet., 206:382
- Guérineau et al. (1990), Plant Mol Biol, 15:127-136
- Hartley, 1988, J. Mol. Biol., 202:913-915
- Hatwell et al. (1999), The Plant Journal, 20(3):333-342.
- Herrera-Estrella et al. (1983), EMBO J, 2:987-995
- Hiei et al. (1994). The Plant Journal, 6:271-282.
- Hoekema et al. (1983). Nature, 303:179-180.
- Hudspeth et al. (1992), Plant physiology, 98:458-464.
- Imaizumi et al. (1997), Plant Mol Biol, 34(5):701-716
- Ishida et al.(1996), Nature Biotechnology 14 : 754-750
- Kasuga et al. (1999), Nature Biotechnologie, 17:287-291
- Kay et al., (1987) Science, 236:4805
- Klein et al. (1987), Nature 327:70-73
- Kogami et al. (1994, Transgenic Research, 3:287-296
- Ku et al. (1999), Nat Biotechnol, 17(1):76-80
- Laemmli et al. (1970), Nature 227:680-685
- Lepiniec et al. (1992a), Plant Mol Biol, 19(2):339-342
- Lepiniec et al. (1992b), Plant Mol Biol, 21:487-502
- Lipka et al. (1999), Plant Science, 144(2):85-92
- Mc Elroy et al. (1990), Plant Cell,2 :163-171
- Mc Elroy et al. (1991). Molecular and General Genetics, 231(1), 150-160.
- Metcalfe et al. (1996), Analytical chemestry, 38:514-515.
- Morris et al. (1992), Virology, 187 :633
- Neuhaus et al. (1987), Theoretical and applied Genet., 75(1):30-36
- Paul et al. (1992), Plant Molecular Biology, 19(4):611-622
- Pelleschi et al. (1997), Plant Cell Environ, 20:493-503
- Robert et al., 1989, Plant Cell, 1 : 569-578
- Rodriguez-Penagos (1999) J. Plant Physiol. 155(4-5) :631-638
- Sambrook et al. (1989). Molecular cloning, A laboratory manual, 2nd Edition cold spring harbor laboratory press
- Schocher et al. (1986). Biotechnology 4:1093-1096
- Smith et al. (1998), Plant Physiol, 118(1):191-197
- Tagu et al. (1991) Plant Cell Reports 9:688-690
- Taybi et al. (2000), Plant Physiology, 123(4):1471-1482.
- Thomas et al. (1987), Biochem Biophys Res Commun 143(1):170-177
- Vain et al. (1989), Plant Cell tissue and organ Culture 18:143-151
- Vieira et al. (1982), Gene, 19(3):259-268
- Wang et al. (1992), J Biol Chem, 267(24):16759-16762
- Watson et al. (1994), ADN recombinant, Ed. De Boeck Université pp 273-292
- White et al. (1990), Nucleic Acids Research 18:1062
- Zambryski et al. (1989) Cell, 56, 193-201.


## Revendications

**1.** Procédé d'amélioration de la tolérance au stress hydrique chez une plante de type C4, comprenant les étapes consistant à :

- introduire dans au moins une cellule de plante de type C4 une cassette d'expression comprenant un acide nucléique codant pour une phosphoénolpyruvate carboxylase (PEPC) de type C4, dont la séquence nucléotidique comprend la séquence SEQ ID No 1 ou une séquence homologue a la séquence SEQ ID No. 1 identique à au moins 95% à la séquence SEQ ID No. 1, sur toute la longueur de SEQ ID No. 1, ledit acide nucléique codant pour un enzyme catalysant la β-carboxylation du phosphoénolpyruvate en présence de bicarbonate et d'un cation bivalent, pour former de l'oxaloacétate et du phosphate inorganique ;
- cultiver la cellule ainsi transformée de manière à régénérer une plante transgénique de type C4 contenant dans son génome ladite cassette d'expression, et surexprimant ladite PEPC.

**2.** Procédé d'amélioration de la tolérance au stress hydrique chez une plante de type C4, selon la revendication 1, **caractérisé en ce que** ledit acide nucléique codant pour la phosphoénolpyruvate carboxylase (PEPC) de type C4 de sorgho présente une séquence nucléotidique comprenant la séquence SEQ ID No. 1 sur toute la longueur de SEQ ID No. 1.

**3.** Procédé selon l'une des revendications 1 et 2, **caractérisé en ce que** la plante de type C4 est le maïs.

**4.** Procédé selon l'une des revendications 1 à 3, comprenant en outre l'identification et la sélection des cellules transformées capables de régénérer des plantes tolérantes au stress hydrique par rapport à une plante non transformée.

**5.** Procédé selon l'une quelconque des revendications 1 à 4, **caractérisé en ce que** la cassette d'expression comprend un promoteur permettant l'expression constitutive ou ciblée de la séquence codant pour la PEPC.

**6.** Procédé selon la revendication 5, **caractérisé en ce que** le promoteur est choisi parmi le promoteur actine-intron, le promoteur HMWG, ou le promoteur PEPC.

**7.** Procédé selon la revendication 6, **caractérisé en ce que** le promoteur est le promoteur de séquence SEQ ID No. 2.

**8.** Plante de type C4 transgénique ou partie de plante de type C4 transgénique, **caractérisée en ce que** la plante est du maïs et comprend dans son génome un transgène comprenant une cassette d'expression intégrée de manière stable comprenant un acide nucléique codant pour la phosphoénolpyruvate carboxylase (PEPC) de type C4 de sorgho, dont la séquence nucléotidique comprend la séquence SEQ ID No 1 ou une séquence homologue à la séquence SEQ ID No 1 identique à au moins 95 % à la séquence SEQ ID No. 1, sur toute la longueur de SEQ ID No. 1, ledit acide nucléique codant pour un enzyme catalysant la β-carboxylation du phosphoénolpyruvate en présence de bicarbonate et d'un cation bivalent, pour former de l'oxaloacétate et du phosphate inorganique.

**9.** Plante de type C4 transgénique ou partie de plante de type C4 transgénique selon la revendication 8, **caractérisée en ce que** ledit acide nucléique codant pour la phosphoénolpyruvate carboxylase (PEPC) de type C4 de sorgho présente une séquence nucléotidique comprenant la séquence SEQ ID No. 1 sur toute la longueur de SEQ ID No. 1.

**10.** Plante selon l'une des revendications 8 ou 9, **caractérisée en ce qu'**il s'agit du maïs.

## Claims

**1.** Method for improving tolerance to water stress in a C4-type plant, comprising the steps consisting in:

- introducing, into at least one C4-type plant cell, an expression cassette comprising a nucleic acid encoding a C4-type phosphoenolpyruvate carboxylase (PEPC), the nucleotide sequence of which comprises the sequence SEQ ID No. 1 or a sequence homologous to the sequence SEQ ID No. 1 which is at least 95% identical to the sequence SEQ ID No. 1, over the entire length of SEQ ID No. 1, said nucleic acid encoding an enzyme which catalyses the β-carboxylation of phosphoenolpyruvate in the presence of bicarbonate and of a divalent cation, so as to form oxaloacetate and inorganic phosphate;
- culturing the cell thus transformed so as to regenerate a C4-type transgenic plant containing said expression

cassette in its genome and overexpressing said PEPC.

2. Method for improving tolerance to water stress in a C4-type plant, according to Claim 1, **characterized in that** said nucleic acid encoding the C4-type phosphoenolpyruvate carboxylase (PEPC) from sorghum has a nucleotide sequence comprising the sequence SEQ ID No. 1 over the entire length of SEQ ID No. 1.

3. Method according to either of Claims 1 and 2, **characterized in that** the C4-type plant is maize.

4. Method according to one of Claims 1 to 3, which also comprises identifying and selecting the transformed cells capable of regenerating plants tolerant to water stress compared with a nontransformed plant.

5. Method according to any one of Claims 1 to 4, **characterized in that** the expression cassette comprises a promoter which allows the constitutive or targeted expression of the sequence encoding the PEPC.

6. Method according to Claim 5, **characterized in that** the promoter is chosen from the actin promoter-intron, the HMWG promoter or the PEPC promoter.

7. Method according to Claim 6, **characterized in that** the promoter is the promoter of sequence SEQ ID No. 2.

8. Transgenic C4-type plant or part of a transgenic C4-type plant, **characterized in that** the plant is maize and comprises, in its genome, a transgene comprising a stably integrated expression cassette comprising a nucleic acid encoding the C4-type phosphoenolpyruvate carboxylase (PEPC) from sorghum, the nucleotide sequence of which comprises the sequence SEQ ID No. 1 or a sequence homologous to the sequence SEQ ID No. 1 which is at least 95% identical to the sequence SEQ ID No. 1, over the entire length of SEQ ID No. 1, said nucleic acid encoding an enzyme which catalyses the z-carboxylation of phosphoenolpyruvate in the presence of bicarbonate and of a divalent cation, so as to form oxaloacetate and inorganic phosphate.

9. Transgenic C4-type plant or part of a transgenic C4-type plant according to Claim 8, **characterized in that** said nucleic acid encoding the C4-type phosphoenolpyruvate carboxylase (PEPC) from sorghum has a nucleotide sequence comprising the sequence SEQ ID No. 1 over the entire length of SEQ ID No. 1.

10. Plant according to either of Claims 8 and 9, **characterized in that** it is maize.

## Patentansprüche

1. Verfahren zur Verbesserung der Toleranz gegenüber Feuchtigkeitsstress bei einer Pflanze vom Typ C4, umfassend die Schritte, welche bestehen aus:

   - Einführen einer Expressionskassette, die Nukleinsäure umfasst, welche für eine Phosphoenolpyruvat-Carboxylase (PEPC) vom Typ C4 kodiert, deren Nukleotidsequenz die Sequenz SEQ ID No. 1 oder eine zu der Sequenz SEQ ID No. 1 homologe Sequenz umfasst, welche zu mindestens 95 % mit der Sequenz SEQ ID No. 1 über die ganze Länge der SEQ ID No. 1 identisch ist, wobei die Nukleinsäure für ein Enzym kodiert, welches die β-Carboxylierung von Phosphoenolpyruvat in Anwesenheit von Bicarbonat und eines zweiwertigen Kations katalysiert, um Oxaloacetat und ein anorganisches Phosphat zu bilden, in mindestens eine Pflanzenzelle vom Typ C4;
   - Kultivieren der so transformierten Zelle auf solche Weise, dass eine transgene Pflanze vom Typ C4 regeneriert wird, die in ihrem Genom die Expressionskassette enthält und die PEPC überexprimiert.

2. Verfahren zur Verbesserung der Toleranz gegenüber Feuchtigkeitsstress bei einer Pflanze vom Typ C4 nach Anspruch 1, **dadurch gekennzeichnet, dass** die Nukleinsäure, die für die Phosphoenolpyruvat-Carboxylase (PEPC) vom Typ C4 von Sorghum eine Nukleotidsequenz aufweist, welche die Sequenz SEQ ID No. 1 über die ganze Länge der SEQ ID No. 1 umfasst.

3. Verfahren nach einem der Ansprüche 1 und 2, **dadurch gekennzeichnet, dass** die Pflanze vom Typ C4 Mais ist.

4. Verfahren nach einem der Ansprüche 1 bis 3, darüber hinaus umfassend die Identifikation und Selektion von transformierten Zellen, welche Pflanzen regenerieren können, die im Verhältnis zu einer nichttransformierten Pflanze

Feuchtigkeitsstress tolerant sind.

5. Verfahren nach irgendeinem der Ansprüche 1 bis 4, **dadurch gekennzeichnet, dass** die Expressionskassette einen Promotor umfasst, der die konstitutive oder gezielte Expression der Sequenz ermöglicht, die für die PEPC kodiert.

6. Verfahren nach Anspruch 5, **dadurch gekennzeichnet, dass** der Promotor ausgewählt ist aus dem Aktin-Intron-Promotor, dem HMWG-Promotor oder dem PEPC-Promotor.

7. Verfahren nach Anspruch 6, **dadurch gekennzeichnet, dass** der Promotor der Promotor der Sequenz SEQ ID No. 2 ist.

8. Transgene Pflanze vom Typ C4 oder Teil einer transgenen Pflanze vom Typ C4, **dadurch gekennzeichnet, dass** die Pflanze Mais ist und in ihrem Genom ein Transgen umfasst, das eine Expressionskassette umfasst, die auf stabile Weise integriert ist und eine Nukleinsäure umfasst, die für Phosphoenolpyruvat-Carboxylase (PEPC) vom Typ C4 von Sorghum kodiert, deren Nukleotidsequenz die Sequenz SEQ ID No. 1 oder eine zu der Sequenz SEQ ID No. 1 homologe Sequenz, die zu mindestens 95 % mit der Sequenz SEQ ID No. 1 über die ganze Länge der SEQ ID No. 1 identisch ist, umfasst, wobei die Nukleinsäure für ein Enzym kodiert, das die β-Carboxylierung von Phosphoenolpyruvat in Anwesenheit von Bicarbonat und eines zweiwertigen Kations katalysiert, um Oxaloacetat und ein anorganisches Phosphat zu bilden.

9. Transgene Pflanze vom Typ C4 oder Teil einer transgenen Pflanze vom Typ C4 nach Anspruch 8, **dadurch gekennzeichnet, dass** die Nukleinsäure, die für die Phosphoenolpyruvat-Carboxylase (PEPC) vom Typ C4 von Sorghum kodiert, eine Nukleotidsequenz aufweist, welche die Sequenz SEQ ID No. 1 über die ganze Länge der SEQ ID No. 1 umfasst.

10. Pflanze nach einem der Ansprüche 8 oder 9, **dadurch gekennzeichnet, dass** es sich um Mais handelt.

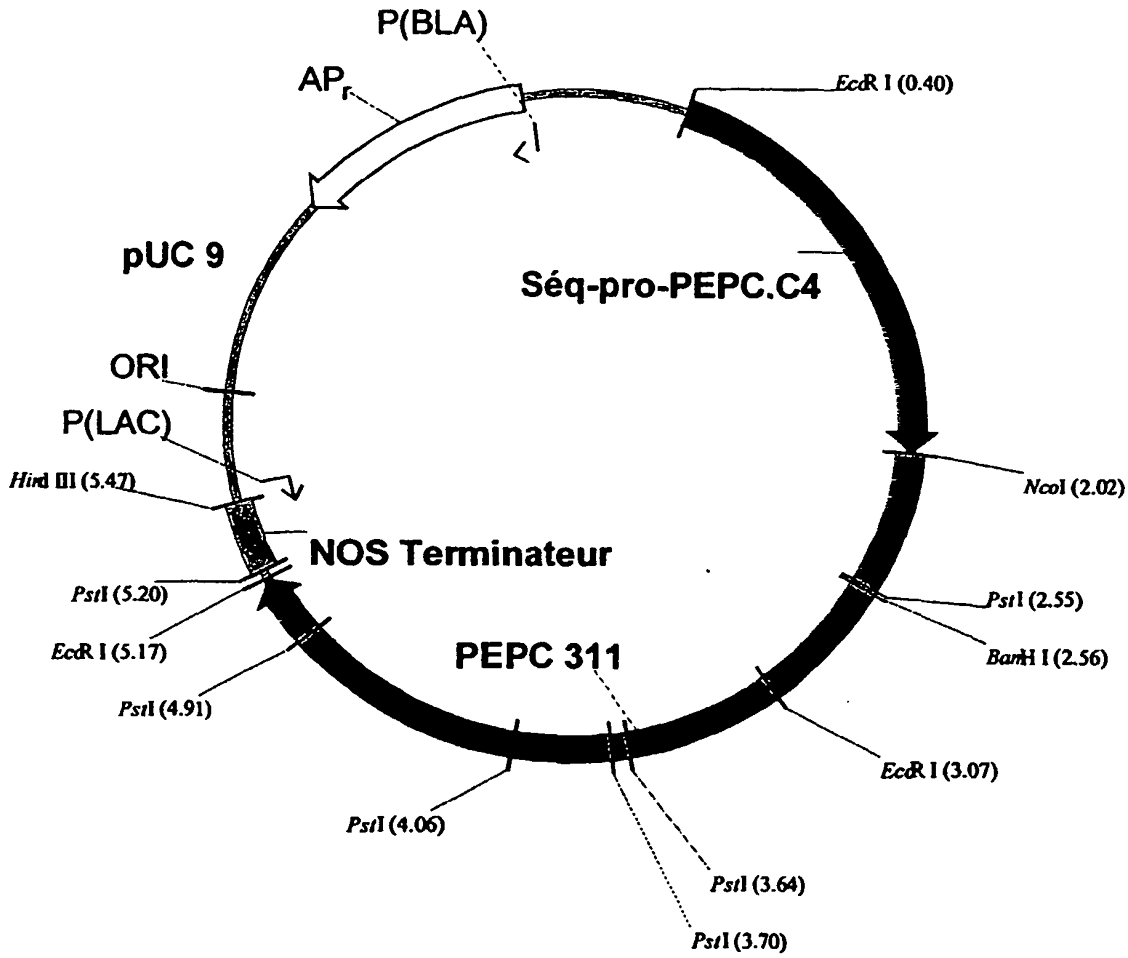
P(BLA)
APr
EcoR I (0.40)
pUC 9
Séq-pro-PEPC.C4
ORI
P(LAC)
Hind III (5.47)
NcoI (2.02)
NOS Terminateur
PstI (5.20)
PstI (2.55)
EcoR I (5.17)
BamH I (2.56)
PEPC 311
PstI (4.91)
EcoR I (3.07)
PstI (4.06)
PstI (3.64)
PstI (3.70)

**Figure 1**

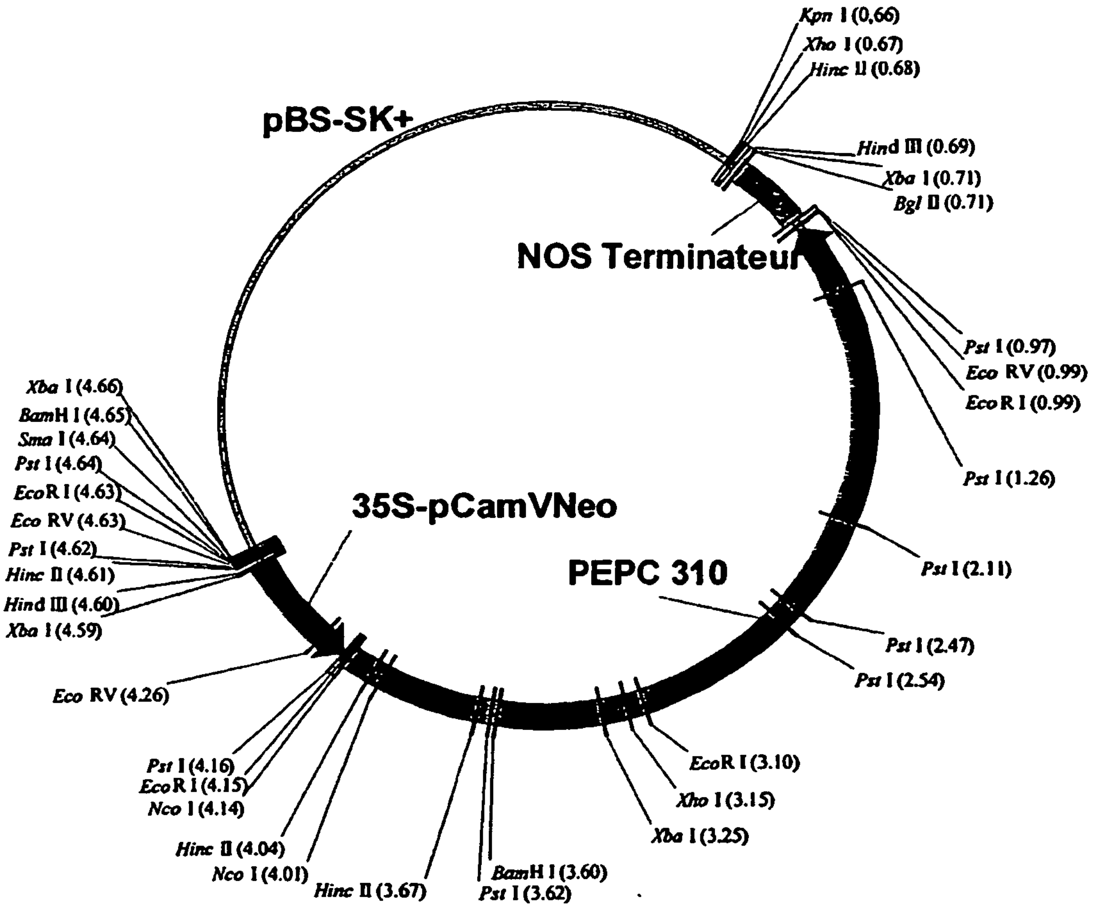
Kpn I (0,66)
Xho I (0.67)
Hinc II (0.68)
Hind III (0.69)
Xba I (0.71)
Bgl II (0.71)
pBS-SK+
NOS Terminateur
Pst I (0.97)
Eco RV (0.99)
EcoR I (0.99)
Pst I (1.26)
Xba I (4.66)
BamH I (4.65)
Sma I (4.64)
Pst I (4.64)
EcoR I (4.63)
Eco RV (4.63)
Pst I (4.62)
Hinc II (4.61)
Hind III (4.60)
Xba I (4.59)
35S-pCamVNeo
PEPC 310
Pst I (2.11)
Pst I (2.47)
Pst I (2.54)
Eco RV (4.26)
Pst I (4.16)
EcoR I (4.15)
Nco I (4.14)
Hinc II (4.04)
Nco I (4.01)
Hinc II (3.67)
BamH I (3.60)
Pst I (3.62)
EcoR I (3.10)
Xho I (3.15)
Xba I (3.25)

**Figure 2**

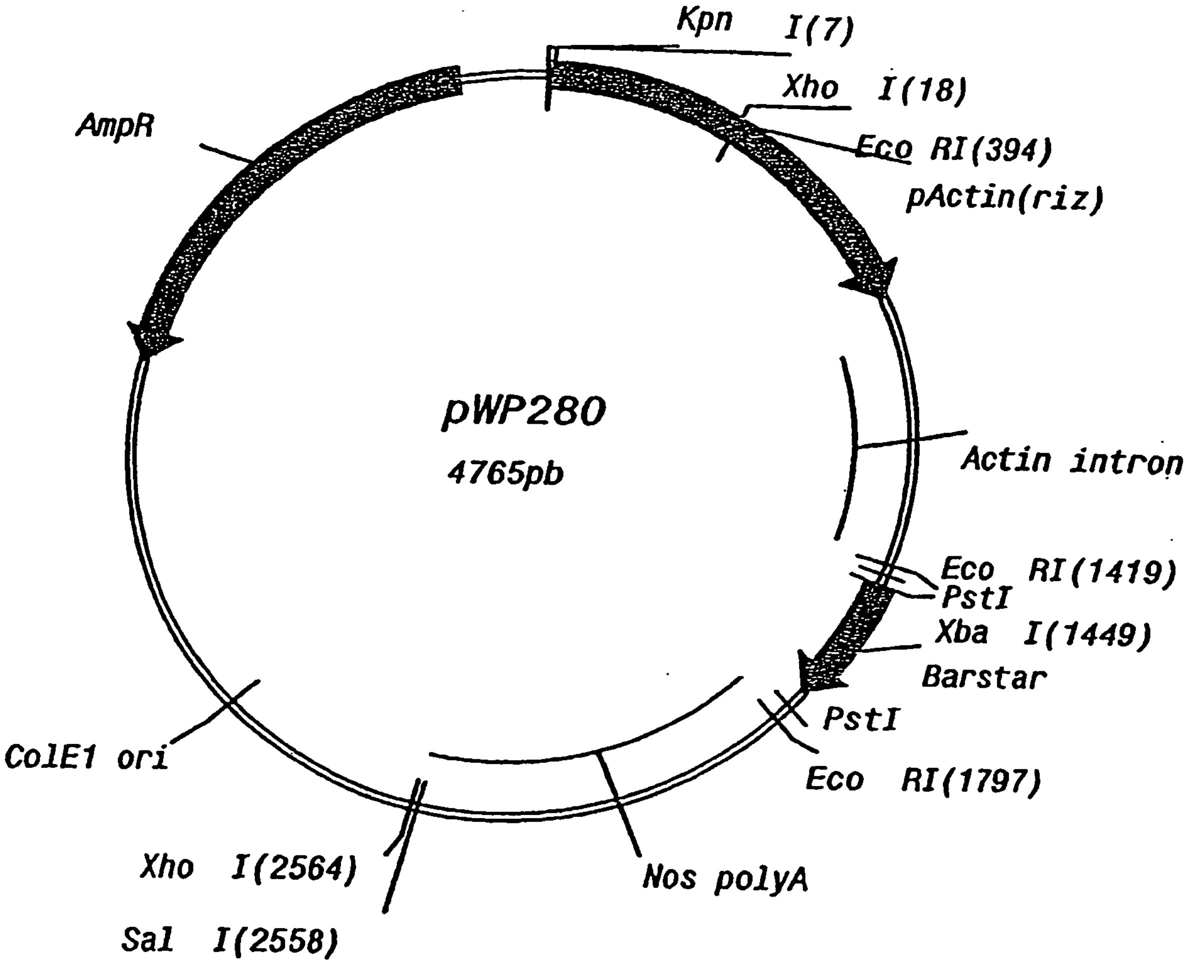
Kpn I(7)
Xho I(18)
Eco RI(394)
pActin(riz)
AmpR
pWP280
4765pb
Actin intron
Eco RI(1419)
PstI
Xba I(1449)
Barstar
PstI
Eco RI(1797)
ColE1 ori
Xho I(2564)
Sal I(2558)
Nos polyA

**Figure 3**

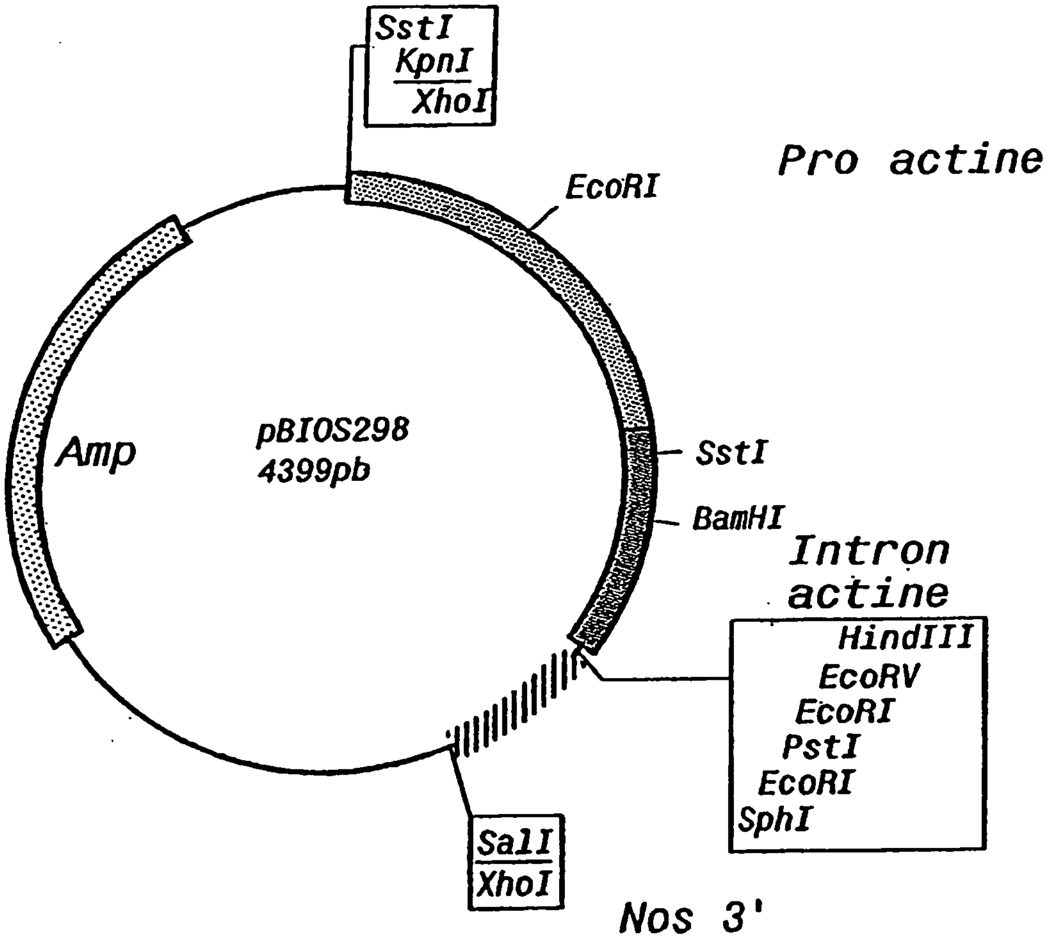
SstI
KpnI
XhoI
Pro actine
EcoRI
Amp
pBIOS298
4399pb
SstI
BamHI
Intron
actine
HindIII
EcoRV
EcoRI
PstI
EcoRI
SphI
SalI
XhoI
Nos 3'

Figure 4

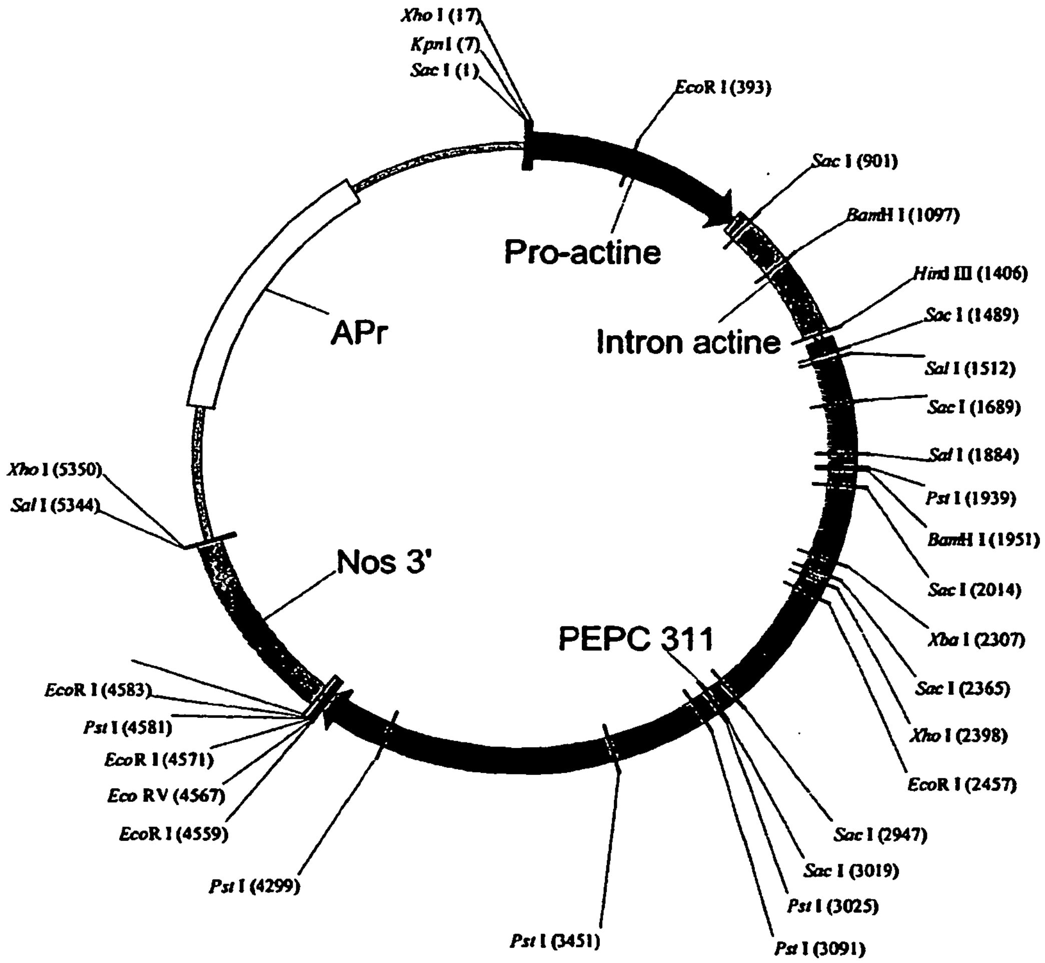
Xho I (17)
Kpn I (7)
Sac I (1)
EcoR I (393)
Sac I (901)
BamH I (1097)
Hind III (1406)
Sac I (1489)
Sal I (1512)
Sac I (1689)
Sal I (1884)
Pst I (1939)
BamH I (1951)
Sac I (2014)
Xba I (2307)
Sac I (2365)
Xho I (2398)
EcoR I (2457)
Sac I (2947)
Sac I (3019)
Pst I (3025)
Pst I (3091)
Pst I (3451)
Pst I (4299)
EcoR I (4559)
Eco RV (4567)
EcoR I (4571)
Pst I (4581)
EcoR I (4583)
Sal I (5344)
Xho I (5350)
Pro-actine
Intron actine
APr
Nos 3'
PEPC 311

**Figure 5**

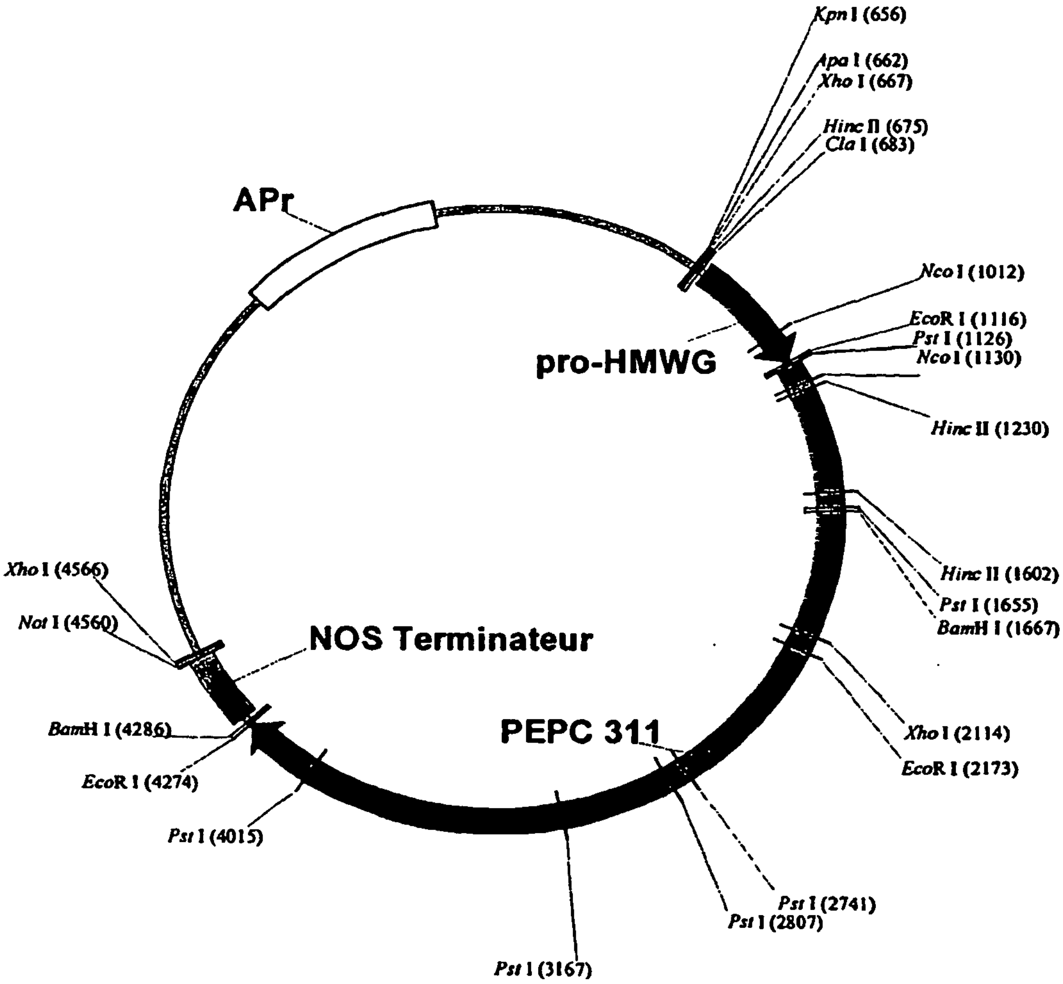
Kpn I (656)
Apa I (662)
Xho I (667)
Hinc II (675)
Cla I (683)
APr
Nco I (1012)
EcoR I (1116)
Pst I (1126)
Nco I (1130)
pro-HMWG
Hinc II (1230)
Hinc II (1602)
Pst I (1655)
BamH I (1667)
Xho I (4566)
Not I (4560)
NOS Terminateur
PEPC 311
Xho I (2114)
EcoR I (2173)
BamH I (4286)
EcoR I (4274)
Pst I (4015)
Pst I (2741)
Pst I (2807)
Pst I (3167)

**Figure 6**

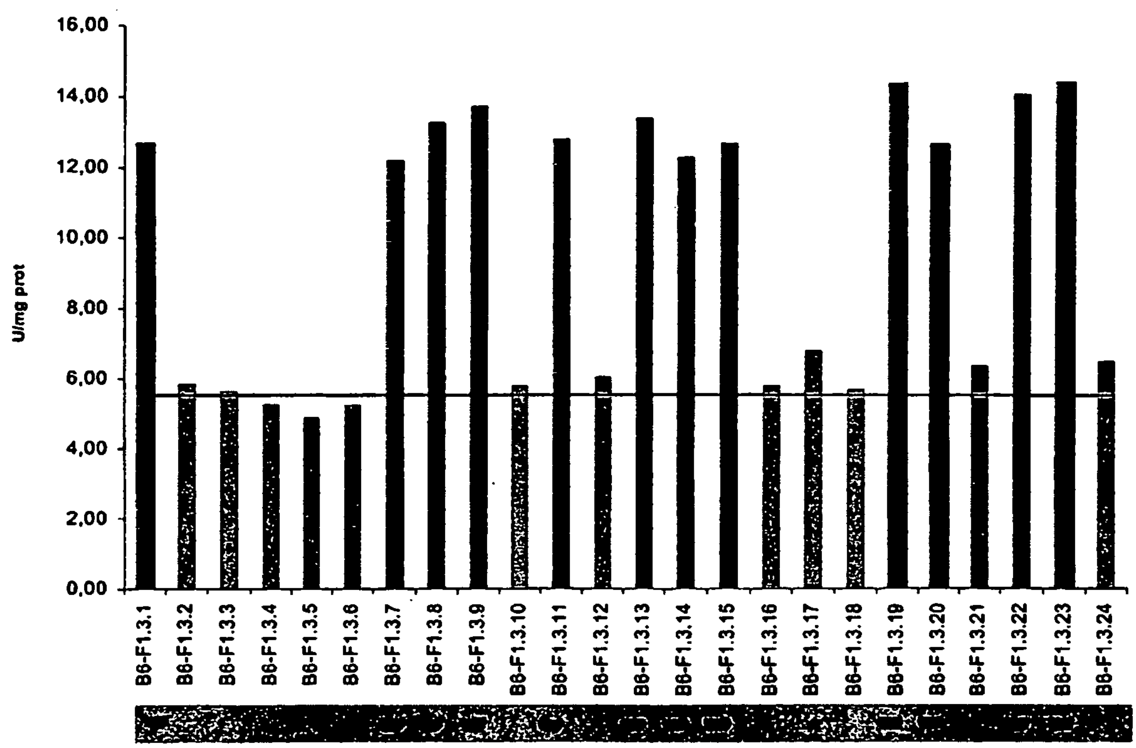
16,00
14,00
12,00
10,00
8,00
6,00
4,00
2,00
0,00
U/mg prot
B6-F1.3.1
B6-F1.3.2
B6-F1.3.3
B6-F1.3.4
B6-F1.3.5
B6-F1.3.6
B6-F1.3.7
B6-F1.3.8
B6-F1.3.9
B6-F1.3.10
B6-F1.3.11
B6-F1.3.12
B6-F1.3.13
B6-F1.3.14
B6-F1.3.15
B6-F1.3.16
B6-F1.3.17
B6-F1.3.18
B6-F1.3.19
B6-F1.3.20
B6-F1.3.21
B6-F1.3.22
B6-F1.3.23
B6-F1.3.24

**Figure 7**

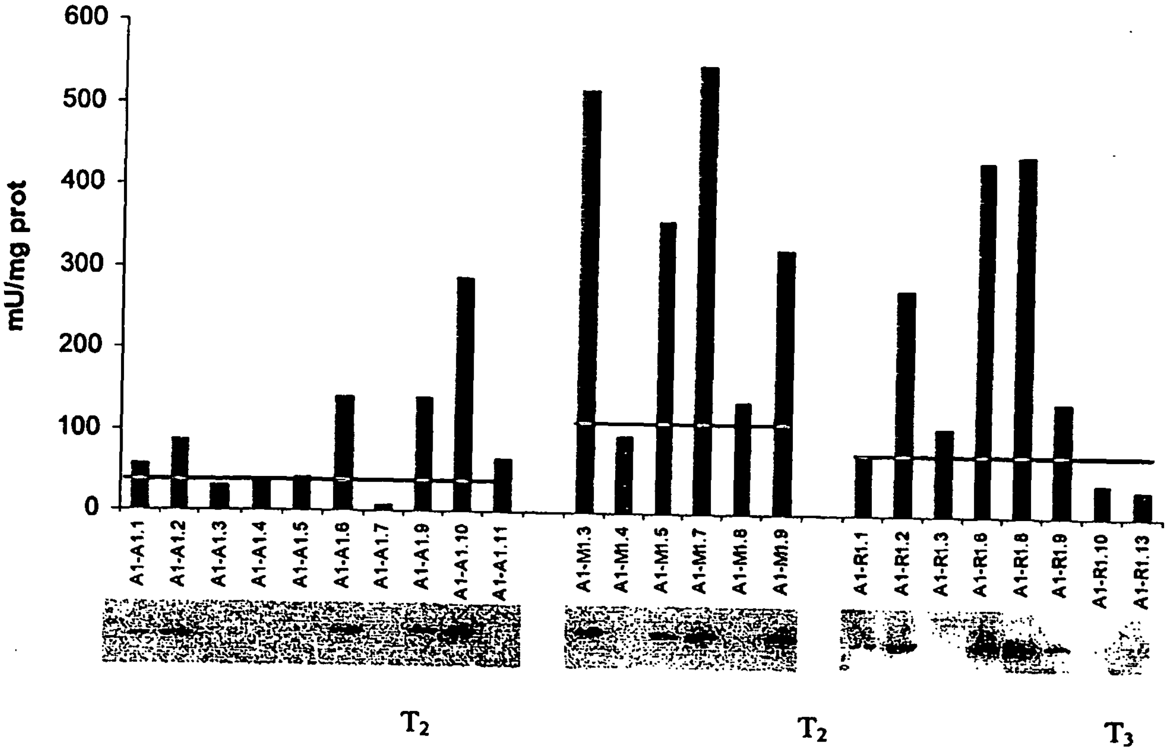
600
500
400
300
200
100
0
mU/mg prot
A1-A1.1
A1-A1.2
A1-A1.3
A1-A1.4
A1-A1.5
A1-A1.6
A1-A1.7
A1-A1.9
A1-A1.10
A1-A1.11
A1-M1.3
A1-M1.4
A1-M1.5
A1-M1.7
A1-M1.8
A1-M1.9
A1-R1.1
A1-R1.2
A1-R1.3
A1-R1.6
A1-R1.8
A1-R1.9
A1-R1.10
A1-R1.13
$T_2$
$T_2$
$T_3$

**Figure 8**

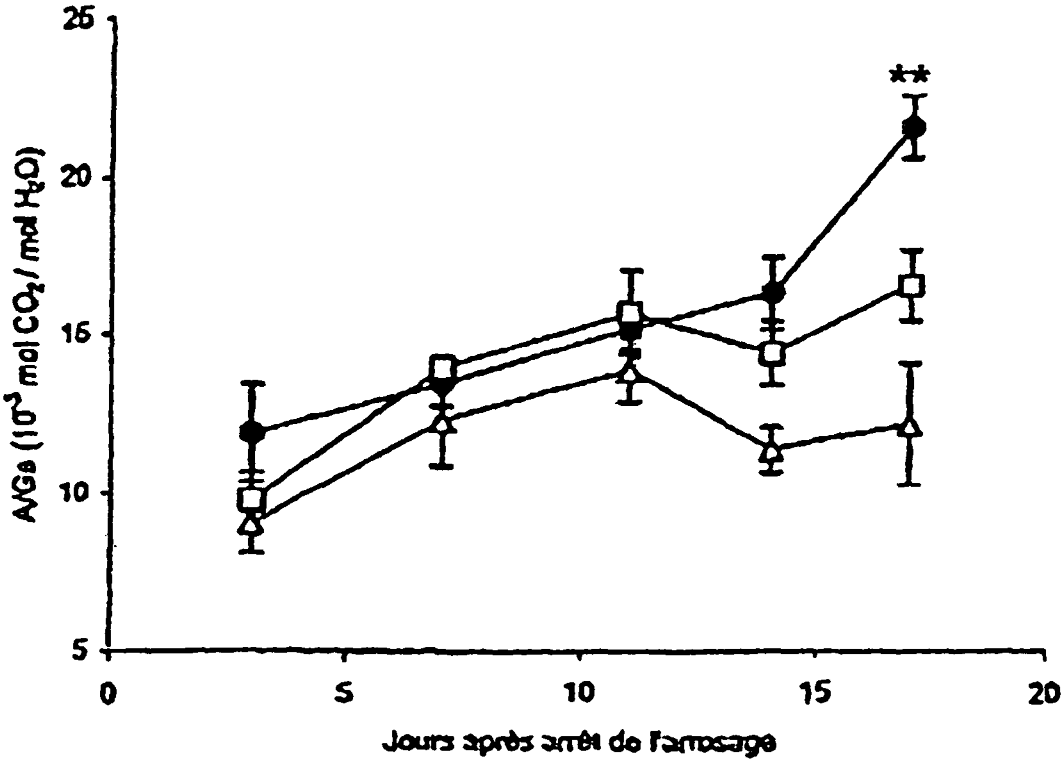
25
20
15
10
5
0
5
10
15
20
**
A/Gs (10⁻³ mol CO2 / mol H2O)
Jours après arrêt de l'arrosage

Figure 9

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 212649 A
- WO 0028017 A
- EP 916725 A
- JP 9292468 A
- JP 9107975 B
- WO 0008187 A
- US 4683202 A
- WO 9849316 A
- US 4407956 A
- EP 672752 A
- FR 0104602
- FR 0114822


### Littérature non-brevet citée dans la description


- **Smith ; Waterman.** *Ad. App. Math.,* 1981, vol. 2, 482
- **Wunsch.** *J. Mol. Biol.,* 1970, vol. 48, 443
- **Lipman.** *Proc. Natl. Acad. Sci. USA,* 1988, vol. 85, 2444
- **Sambmok et al.** Molecular Cloning, A laboratory manual. Cold Spring Harbor laboratory Press, 1989, 9.54-9.62
- **Walker et al.** *Nucleic Acids Res.,* 1992, vol. 20, 1691
- **Kwoh et al.** *Proc. Natl. Acad. Sci. USA,* 1989, vol. 86, 1173
- **Guatelli et al.** *Proc. Natl. Acad. Sci. USA,* 1990, vol. 87, 1874
- **Kievitis et al.** *J. Virol. Methods,* 1991, vol. 35, 273
- **Landegren et al.** *Science,* 1988, vol. 241, 1077
- **Segev ; Kessler C.** Springer Verlag. 1992, 197-205
- **Duck et al.** *Biotechniques,* 1990, vol. 9, 142
- **Miele et al.** *J. Mol. Biol.,* 1983, vol. 171, 281
- **Moss B.** *PNAS USA,* 1989, vol. 86, 6126-6130
- *Virology,* vol. 154, 9-20
- **Macejack, D.G. ; P. Samow.** *Nature,* 1991, vol. 353, 90-94
- **Jobling, S.A. ; Gehrke, L.** *Nature,* 1987, vol. 325, 622-625
- **Gallie, D.R. et al.** *Molecular Biology of RNA,* 1989, 237-256
- Recombinant DNA Part D. *Methods in Enzymology,* 1987, vol. 153
- **Hohn et al.** Molecular Biology of Plant Tumors. Academic Press, 1982, 549-560
- Molecular Cloning : A Laboratory Manual. **Sambrook et al.** Molecular cloning. Cold Spring Harbor Laboratory Press, 1989
- **Hartley.** *J. Mol. Biol.,* 1988, vol. 202, 913-915
- **Guérineau et al.** *Plant Mol. Biol.,* 1990, vol. 15, 127-136
- **Dale et al.** *Gene,* 1990, vol. 91, 79-85
- **Mc Elroy et al.** *Mol. Gen. Genet.,* 1991, vol. 231, 150-160
- Maize Handbook. 1994, 665-671
- *Methods of Molecular Biology: Plant gene transfer and expression protocols,* 1995, vol. 49, 113-123
- **An et al.** *Plant Physiol,* vol. 81, 86-91
- **Armstrong et al.** *The Maize Handbook, 665-671,* 1994
- **Bandurski ; Greiner.** *J. Biol. Chem.,* 1953, vol. 204, 781-786
- **Barcelo et al.** *Methods of Molecular Biology,* 1995, vol. 49, 113-123
- **Bechtold et al.** *Comptes rendus Académie des Sciences Paris Serie,* 1993, vol. 3 (316), 1194-1199
- **Blechl et al.** *Nat Biotechnol,* 1996, vol. 14 (7), 875-879
- **Bligh et al.** *Canadian journal ofbiochemestry and physiology,* 1959, vol. 37, 911917
- **Callis.** *Genes Dev.,* 1987, vol. 1, 1183
- **Cao et al.** *Plant Cell Reports,* 1992
- **Chupeau et al.** *Biotechnology,* 1989, vol. 7 (5), 503-508
- **Contour-Ansel et al.** *J. Agron. & Crop Sci.,* 1996, vol. 176 (1), 59-69
- **Coursol et al.** *The Plant Journal,* 2000, vol. 23 (4), 497-506
- **Cretin et al.** *Nucleic Acids Res,* 1990, vol. 18 (3), 658
- **Cretin et al.** *Gene,* 1991, vol. 99, 87-94
- **Cretin et al.** *Plant Molecular Biology,* 1991, vol. 17, 83-88

- **Depicker et al.** *J. Mol. Appl.Genet.,* 1982, vol. 1, 561-573
- **Finer et al.** *Plant Cell Report,* 1992, vol. 11, 323-328
- **Franck et al.** *Cell,* 1980, vol. 21, 285-294
- **Fromm M et al.** *Biotechnology,* 1990, vol. 8, 833-839
- **Gehlen et al.** *Plant Mol Biol,* 1996, vol. 32 (5), 831-848
- **Gonzalez et al.** *Plant physiology,* 1998, vol. 116, 1249-1258
- **Guerche et al.** *Mol. Gen. Genet.,* 1987, vol. 206, 382
- **Guérineau et al.** *Plant Mol Biol,* 1990, vol. 15, 127-136
- **Hatwell et al.** *The Plant Journal,* 1999, vol. 20 (3), 333-342
- **Herrera-Estrella et al.** *EMBO J,* 1983, vol. 2, 987-995
- **Hiei et al.** *The Plant Journal,* 1994, vol. 6, 271-282
- **Hoekema et al.** *Nature,* 1983, vol. 303, 179-180
- **Hudspeth et al.** *Plant physiology,* 1992, vol. 98, 458-464
- **Imaizumi et al.** *Plant Mol Biol,* 1997, vol. 34 (5), 701-716
- **Ishida et al.** *Nature Biotechnology,* 1996, vol. 14, 754-750
- **Kasuga et al.** *Nature Biotechnologie,* 1999, vol. 17, 287-291
- **Kay et al.** *Science,* 1987, vol. 236, 4805
- **Klein et al.** *Nature,* 1987, vol. 327, 70-73
- **Kogami et al.** *Transgenic Research,* 1994, vol. 3, 287-296
- **Ku et al.** *Nat Biotechnol,* 1999, vol. 17 (1), 76-80
- **Laemmli et al.** *Nature,* 1970, vol. 227, 680-685
- **Lepiniec et al.** *Plant Mol Biol,* 1992, vol. 19 (2), 339-342
- **Lepiniec et al.** *Plant Mol Biol,* 1992, vol. 21, 487-502
- **Lipka et al.** *Plant Science,* 1999, vol. 144 (2), 85-92
- **Mc Elroy et al.** *Plant Cell,* 1990, vol. 2, 163-171
- **Mc Elroy et al.** *Molecular and General Genetics,* 1991, vol. 231 (1), 150-160
- **Metcalfe et al.** *Analytical chemestry,* 1996, vol. 38, 514-515
- **Morris et al.** *Virology,* 1992, vol. 187, 633
- **Neuhaus et al.** *Theoretical and applied Genet.,* 1997, vol. 75 (1), 30-36
- **Paul et al.** *Plant Molecular Biology,* 1992, vol. 19 (4), 611-622
- **Pelleschi et al.** *Plant Cell Environ,* 1997, vol. 20, 493-503
- **Robert et al.** *Plant Cell,* 1989, vol. 1, 569-578
- **Rodriguez-Penagos.** *J. Plant Physiol.,* 1999, vol. 155 (4-5), 631-638
- **Sambrook et al.** Molecular cloning, A laboratory manual. cold spring harbor laboratory press, 1989
- **Schocher et al.** *Biotechnology,* 1986, vol. 4, 1093-1096
- **Smith et al.** *Plant Physiol,* 1998, vol. 118 (1), 191-197
- **Tagu et al.** *Plant Cell Reports,* 1991, vol. 9, 688-690
- **Taybi et al.** *Plant Physiology,* 2000, vol. 123 (4), 1471-1482
- **Thomas et al.** *Biochem Biophys Res Commun,* 1987, vol. 143 (1), 170-177
- **Vain et al.** *Plant Cell tissue and organ Culture,* 1989, vol. 18, 143-151
- **Vieira et al.** *Gene,* 1982, vol. 19 (3), 259-268
- **Wang et al.** *J Biol Chem,* 1992, vol. 267 (24), 16759-16762
- **Watson et al.** ADN recombinant. De Boeck Université, 1994, 273-292
- **White et al.** *Nucleic Acids Research,* 1990, vol. 18, 1062
- **Zambryski et al.** *Cell,* 1989, vol. 56, 193-201